(19)

Europäisches Patentamt
European Patent Office
Office européen des brevets

(11) **EP 4 113 538 A1**

(12) **EUROPEAN PATENT APPLICATION**
published in accordance with Art. 153(4) EPC

(43) Date of publication:
**04.01.2023 Bulletin 2023/01**

(21) Application number: **21748230.6**

(22) Date of filing: **29.01.2021**

(51) International Patent Classification (IPC):
**G16Z 99/00** (2019.01)          **G16H 30/00** (2018.01)

(52) Cooperative Patent Classification (CPC):
**G16H 10/40; G16H 30/00; G16Z 99/00**

(86) International application number:
**PCT/JP2021/003206**

(87) International publication number:
**WO 2021/153721 (05.08.2021 Gazette 2021/31)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.01.2020   JP 2020013646**

(71) Applicant: **Cardio Flow Design Inc.
Tokyo 102-0082 (JP)**

(72) Inventors:
• **SAKAJO, Takashi
Kyoto-shi, Kyoto 606-8501 (JP)**
• **ITATANI, Keiichi
Kyoto-shi, Kyoto 602-8566 (JP)**

(74) Representative: **Plasseraud IP
66, rue de la Chaussée d'Antin
75440 Paris Cedex 09 (FR)**

(54) **FLOW PATTERN WORD REPRESENTATION DEVICE, WORD REPRESENTATION METHOD, AND PROGRAM**

(57)   This word representation device comprises a storage unit and a word representation generation unit. With respect to the streamline structures constituting flow patterns, the storage unit stores the correspondence between each streamline structure and the characters thereof. The word representation generation unit is provided with a root determination means, a tree representation configuration means, and a COT representation generation means. The root determination means determines a root for a given flow pattern. The tree representation configuration means configures a tree representation for the given flow pattern by repeatedly executing a process of extracting the streamline structure of the flow pattern, adding characters to the streamline structure, and deleting the extracted streamline structure, until the root is reached. The COT representation generation means converts the tree representation configured by the tree representation configuration means to a COT representation and generates a word representation of a given flow pattern. The flow constituting the flow pattern includes a flow generated by moving physical boundaries.

[Figure 18]

EP 4 113 538 A1

**Description**

TECHNICAL FIELD

The present invention relates to a word representation device, a word representation method and a program for a flow pattern.

BACKGROUND ART

A technology for giving many-to-one word representation (maximum word representation) for flow patterns of an incompressible fluid on a surface, and a method for designing the shape of structures in fluids using word representation and regular representation are disclosed in Patent Document 1. In addition, a technique for giving a word representation (regular representation) from a graph representation with a one-to-one correspondence to flow patterns for flow structures of an incompressible fluid on a surface is disclosed in Patent Document 2. Moreover, regarding the blood flow in the heart, a technique for acquiring the blood flow velocity distribution in the heart from the ultrasonic measurement cross-sectional plane and for displaying the streamline is disclosed in Patent Document 3, and a technique for displaying a streamline in a lumen or a cross-sectional plane from the cardiac MRI is disclosed in Patent Document 4.

PRIOR ART

Patent Document

Patent Document 1: International Publication No. 2014/041917

Patent Document 2: International Publication No. 2016/072515

Patent Document 3: International Publication No. 2013/077013

Patent Document 4: International Publication No. 2014/185521

SUMMARY

The aforementioned technique can give an accurate word representation to the flow pattern of an incompressible fluid on a surface, but it is not possible to represent a general flow pattern including compressible fluid part, passing through the cross-sectional plane, especially one with a source or sink in the measurement surface. In addition, because the word representation by above techniques is constructed based on the slip boundary condition on the physical boundaries, it is not possible to give a word representation for an internal flow wherein the surrounding wall compresses and expels, or the wall sucks the flow with its expansion, where the physical boundary becomes the source of the flow, for example, in a bag like pump like a heart.

The present disclosure has been made to solve these problems, and an its objective is to give a word representation for a flow phenomenon in which moving physical boundaries cause a flow, and to give a word representation to a two-dimensional measurement cross-sectional plane of a three-dimensional flow. Its application is to classify the flow pattern of the vortex of blood flow in the heart acquired in medical images such as ultrasonic waves and cardiac MRI, and to give a word representation to the flow pattern.

In order to solve the above problems, an embodiment of the present disclosure is a device for generating a word representation device of a streamline structure of a flow pattern in a two-dimensional region, including a storage unit, a word representation generation unit. The storage unit stores the correspondence relationship between each structure and its characters corresponding to a plurality of structures constituting the flow pattern, and the word representation generation unit includes a root determination means, a tree representation construction means, and a COT representation (COT: partially Cyclically Ordered Tree Representation) generation means. The root determining means determines the root of the given flow pattern. The tree representation constructing means extracts the structure of the given flow pattern, assigns characters in the extracted structure based on the correspondence stored in the storage unit, and constructs the tree representation of a given flow pattern by repeatedly executing the process of giving and deleting the extracted streamline structure from the innermost side of the flow pattern until the root is reached. The COT representation generation means converts the tree representation constructed by the tree representation constructing means into a COT representation to generate a word representation of a given flow pattern, and the flows constructing a flow pattern includes the flow generated by the moving physical boundaries. Here, the "two-dimensional region" refers to a region on a surface that forms a region of interest (ROI). The "physical boundary" is the boundary curve between the two-dimensional region and the external region. "Moving physical boundary" means that this boundary moves with time. For example, when the "two-dimensional region" is the longitudinal cross-sectional plane of the left ventricle, the "physical boundary" is the contour of the wall surrounding the left ventricle in the cross-sectional plane, and the "moving physical boundary" means that the contour of this wall contracts and expands with motion.

Another embodiment of the present disclosure is a method for generating word representation. This method is a word representation method for the streamline structure of a flow pattern in a two-dimensional region, executed by a computer equipped with a storage unit and a word representation generation unit. The storage unit stores the correspondence relationship between each structure and its characters corresponding to a plurality of structures constituting the flow pattern, and the word representation generation unit executes a root determination step, a tree representation construction step, and a COT representation generation step. The root determination step determines the root of the given flow pattern. The tree representation construction step extracts the streamline structure of the given flow pattern, assigns characters in the extracted structure based on the correspondence stored in the storage unit, and constructs a tree representation of a given flow pattern by repeating the process of adding characters to the line structure and deleting the extracted streamline structure from the innermost part of the flow pattern until the root is reached. The COT representation generation step converts the tree representation composed of the tree representation composition steps into a COT representation to generate a word representation of a given flow pattern. Here the flows constructing a flow pattern includes the flow generated by the moving physical boundaries.

Furthermore, another embodiment of the present disclosure is a program. This program that runs on a computer equipped with a storage unit and a word representation generation unit to execute processing. The storage unit stores the correspondence between each streamline structure and its characters with respect to a plurality of streamline structures constituting the flow pattern and the word representation generation unit executes a root determination step, a tree representation construction step, and a COT representation generation step. The root determination step determines the root of the given flow pattern, The tree representation construction step extracts the streamline structure of the given flow pattern assigns characters in the extracted structure based on the correspondence stored in the storage unit, and construct a tree representation of a given flow pattern by repeating the process of adding characters to the line structure and deleting the extracted streamline structure from the innermost part of the flow pattern until the root is reached. The COT representation generation step converts the tree representation composed of the tree representation composition steps into a COT representation to generate a word representation of a given flow pattern. Here, a flow constructing a flow pattern includes the flow generated by the moving physical boundaries.

It should be noted that any combination of the above components, or any mutual replacement of the components and representations of the present disclosure between methods, devices, programs, temporary or non-temporary storage media in which programs are recorded, systems, and the like are also valid as an embodiment of the present disclosure.

In addition, another embodiment of the present disclosure is a device. This device is a word representation device that represents the streamline structure of a flow pattern in a two-dimensional region, and includes a storage unit and a word representation generation unit. The storage unit stores the correspondence relationship between each structure and its characters with respect to a plurality of structures constituting the flow pattern, and the word representation generation unit includes a root determination method, a tree representation construction means, and a COT representation generation means. The root determining means determines the root of the given flow pattern. The tree representation construction means extracts the structure of the given flow pattern, assigns characters in the extracted structure based on the correspondence stored in the storage unit, and constructs a given flow pattern by repeatedly executing the process of giving and deleting the extracted streamline structure from the innermost side of the flow pattern until the root is reached. The COT representation generation means converts the tree representation constructed by the tree representation constructing means into a COT representation to generate a word representation of a given flow pattern. Here, the flows constructing a flow pattern includes the flow generated by the moving physical boundaries.

According to the present disclosure, it is possible to give a word representation to the intra-cross-sectional plane flow of a three-dimensional fluid surrounded by physical boundaries with movement, for example, to give a word representation to the flow pattern of a vortex generated in the heart.

BRIEF DESCRIPTION OF DRAWINGS

FIG. **1** is an explanatory view showing the periodic motion of blood flow, blood pressure, and heart valves during contraction and relaxation of the heart (left ventricle) in normal anatomy (situs solitus, concordant atrioventricular connection, normal great vessel position).
FIG. **2** is a diagram showing a slidable saddle and a slidable $\partial$-saddle. (a) and (b) indicate a sliding saddle. (c) and

(d) indicate slidable $\partial$-saddle.

FIG. **3** is a diagram showing a classification of orbit structures in a complement of Bd (v), a set of boundary orbits of v: flow of finite type. (a) shows an open box whose orbit space is an open interval. (b) shows an open annulus whose orbit space is a circle. (c) shows an open annulus whose orbit space is an open interval.

FIG. **4** is a diagram showing orbit structures (two-dimensional structure) in (Bd (v)) c. (a) shows structures b~±. (b) shows the structure $b_+$ .

FIG. **5** is a diagram showing the root structure on a spherical surface S. (a) shows the structures $\sigma_{\varphi\pm}$, $\sigma_{\varphi-\pm 0}$, $\sigma_{\varphi\sim\pm\pm}$, and $\sigma_{\varphi\sim\pm\mp}$. (b) shows the structure $\beta_{\varphi\pm}$. (c) shows the structure $\beta_{\varphi 2}$.

FIG. **6** is a diagram showing samples of a zero-dimensional point structure and a one-dimensional structure of the flow of Bd (v). (a) shows the structure $\beta_+$ as a one-dimensional structure. (b) shows the structures $\sigma_\pm$, $\sigma_{\sim\pm 0}$, $\sigma_{\sim\pm\pm}$, and $\sigma_{\sim\pm\mp}$ as a zero-dimensional point structure. (c) shows the structures $p_{\sim\pm}$ as a one-dimensional structure. (d) shows the structure $p_+$ as a one-dimensional structure.

FIG. **7** is a diagram showing a one-dimensional structure of the (S1) series. (a) shows the structure $a_+$. (b) shows the structure $q_+$.

FIG. **8** is a diagram showing a one-dimensional structure of the (S2) series. (a) shows the structure b±±. (b) shows the structure $b_{\pm\mp}$.

FIG. **9** is a diagram showing a one-dimensional structure of the (S4) series. (a) shows the structure $\beta_+$. (b) shows the structure $c_+$. (c) shows the structure $c_{2+}$.

FIG. **10** is a diagram showing a one-dimensional structure of the (S5) series. (a) shows the structure $a_2$. (b) shows the structure $\gamma_{\varphi\sim\pm}$. (c) shows the structure $\gamma_{\sim\pm-}$. (d) shows the structure $\gamma_{\sim\pm+}$.

FIG. **11** is a diagram showing a one-dimensional structure of the (S3) series. (a) shows the structures $a_{\sim\pm}$. (b) shows the structures $q_{\sim\pm}$.

FIG. **12** is a diagram showing generation of an n-bundled ss-saddle.

FIG. **13** is a diagram showing samples of ss-components and ss-separatrix connecting to them.

FIG. **14** is a diagram showing three types of non-trivial limit cycles appearing in a flow of finite type.

FIG. **15** is a diagram showing a root structure s $_{\varphi n}$. (a) is a reprint of FIG. 4. (b) shows the structure $s_{\varphi 0}$. (c) shows the structure $s_{\varphi 1}$. (d) shows the structure $s_{\varphi n}$ ($n \geqq 2$).

FIG. **16** is a diagram showing a zero-dimensional structure: $\infty_{\sim\pm}$.

FIG. **17** is an explanatory diagram of a tree representation.

FIG. **18** is a functional block diagram of a word expression device according to the first embodiment.

FIG. **19** is a functional block diagram of a word expression device according to the second embodiment.

FIG. **20** is a flow chart of a word expression method according to the third embodiment.

FIG. **21** is a flow chart showing the process of step N1.

FIG. **22** is a flow chart showing the process of step N2.

FIG. **23(a)** is a flow chart showing the first half of the process of step N3.

FIG. **23(b)** is a flow chart showing the latter half of the process of step N3.

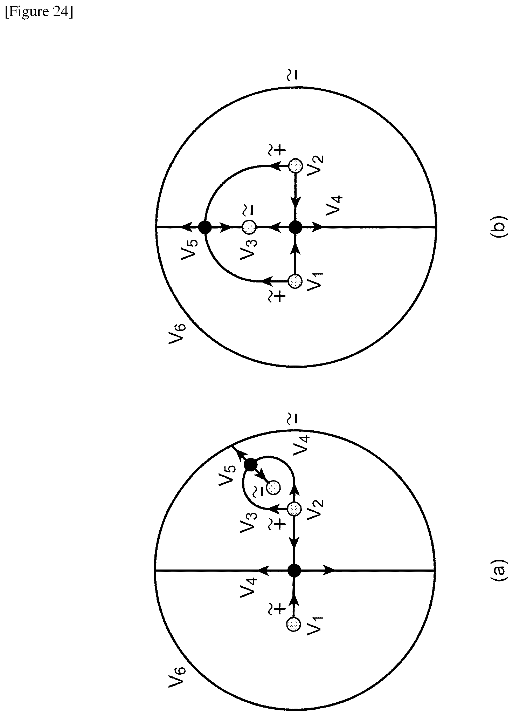

FIG. **24** is a diagram showing an example of a flow pattern having the same COT representation but different streamline topologies.

FIG. **25** is a diagram showing an example of topological preconditioning for intraventricular blood flow image data. (a) shows the flow pattern of the intraventricular blood flow imaging. (b) shows an incomplete topological data structure extracted from (a). (c) shows a consistent topological data structure obtained by topological preconditioning on (b) and adding only one saddle to the outside.

FIG. **26** is a diagram showing how to give a COT expression to the cardiovascular flow of pattern A. (a) shows the flow pattern of the ventricular blood flow imaging. (b) shows a topological data structure extracted from the orbit structure of (a). (c) shows a state where the innermost singular point is removed from (b). (d) shows the orbit structure obtained by removing the structures $a_{\sim-}$ from (c) and by degenerating to the boundary.

FIG. **27** is a diagram showing how to give a COT expression to the cardiovascular system of patterns B, C and D. (a) shows pattern B. (b) shows pattern C. (c) shows pattern D.

FIG. **28** is a visualized streamline of the left ventricular intracardiac blood flow during the early and mid-systolic phase analyzed by echocardiography VFM (vector flow mapping), that is, the phase when the aortic valve begins to open. (a) shows an example of a healthy volunteer, (b) shows an example of a heart failure patient 1, and (c) shows an example of a heart failure patient 2.

EMBODIMENT

The present disclosure will be explained below with reference to each drawing based on a preferred embodiment. In the embodiments and its modification examples, identical or equivalent components and their parts are designated with the same reference symbols, and redundant explanations will be omitted where appropriate. The dimensions of the parts in each drawing are shown enlarged or reduced as appropriate for ease of understanding. Further, in each drawing, some elements that are not important in explaining the form are omitted. Also, in each of the drawings displayed, some elements that are not important in explaining the embodiment are omitted. In addition, terms including ordinal numbers such as first and second are used to describe various components, but these terms are used only for the purpose of distinguishing one component from other components, and the components are not limited by these terms.

[Blood flow in the left ventricle]

The heart ejects blood cyclically. The mechanism is that the myocardium contracts and expands repeatedly, and at the same time, the atrioventricular valve, located on the inlet of the ventricle, and the semilunar valve, located on the outlet, open and close alternately, so that a constant amount of blood is ejected at each heartbeat. In the normal anatomy, the heart has a left ventricle and a right ventricle. Since the present description is an attempt to include the classification of the orbit groups of the intracardiac blood flow obtained from the cardiovascular imaging as clinical examinations, and its discrete combination structure as an application, it will also deal with streamline visualization techniques for intracardiac blood flow.

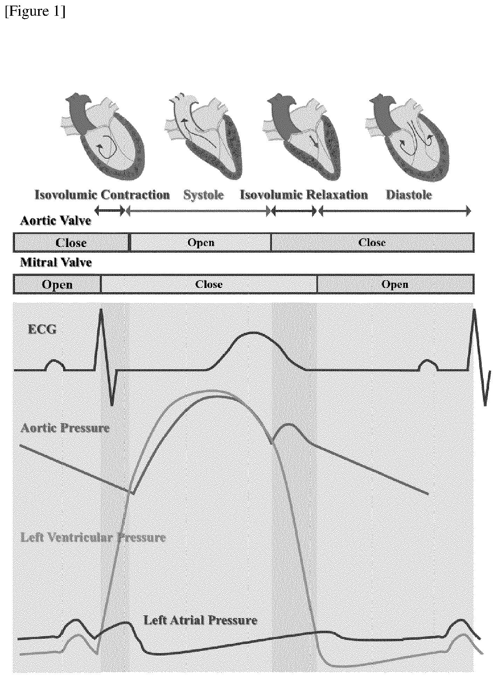

FIG. 1 illustrates the periodic motion of blood flow, blood pressure, and heart valves associated with contraction and relaxation of the heart (left ventricle) in a normal anatomy (situs solitus, concordant atrioventricular connection, and normal great vessel position). Normally in the left ventricle, the mitral valve, the atrioventricular valve on the inlet, and the aortic valve, the semilunar valve at the outlet, repeatedly open and close, and the following four phases are repeated in a single cardiac cycle.

(1) Isovolumic Contraction: The ventricles fill and the mitral valve, an atrioventricular valve, closes. It is a phase when the aortic valve, a semilunar valve, does not yet open, and intracardiac pressure increases while ventricular volume is preserved.

(2) Systole: It is a phase when ventricular pressure exceeds the aortic pressure, the aortic valve opens, the myocardium contracts, the ventricular volume shrinks, and blood is ejected into the aorta as if squeezed out. The left ventricular myocardium not only contracts to simply crush the pouch-like ventricle, but the myocardium twists and contracts in the direction of the fibers.

The systolic phase can be roughly divided into early systole (early systole), when blood flow through the aortic valve is accelerated, the mid systole (mid systole), when a large amount of blood is ejected into the aorta, and the late systole (late systole), when the blood flow through the aortic valve decelerated.

(3) Isovolumic Relaxation: When blood in the ventricle is ejected, the aortic pressure exceeds the intraventricular pressure and the aortic valve closes. It is a phase when the mitral valve does not open yet, the left ventricular myocardium is actively dilated, and the intraventricular pressure decreases while maintaining the volume.

(4) Diastole: It is a phase when the mitral valve opens when the left ventricular pressure drops below the left atrial pressure, and blood flows into the left ventricle. During this phase, the myocardium expands, the volume of the left ventricle increases, and the left ventricle fills.

It is known that opposite to the systole described above, the ventricle not only dilates like a pouch-like shape but also untwists in the direction of the myocardial fibers. In diastole, the rapid inflow phase (rapid filling) when the blood flow through the mitral valve increases with myocardial dilation is followed by the slow inflow phase (late filling) when the velocity of blood flow through the mitral valve decreases. After that, the blood flow through the mitral valve increases again due to the contraction of the left atrium, and this phase is referred to as the atrial contraction period. The subsequent time phase is called end diastole. Also, like systole, the diastole is divided into 3 periods, referred to as early diastole when there is a rapid inflow of blood flow through the mitral valve, mid diastole when the inflow blood flow decelerates, and the flow is relatively static and becomes uniformly slow, and late diastole when re-inflow into the left ventricle due to atrial contraction.

By repeating the above-mentioned cardiac cycle, periodic blood ejection is performed. In the left ventricle structure, the inlet mitral valve and the outlet aortic valve contact with each other side by side by sharing the annulus anatomically. Therefore, the directions of the inflow blood flow and that of outflow are different by approximately 180 degrees. Therefore, the blood flow must turn its direction 180 degrees in the pouch-shaped left ventricle, and a vortex is generated in the chamber. Anatomically, the part where the aortic valve and the mitral valve are connected is called the base, the pouch-like blind end is called the apex, and the part between the base and the apex is called the mid. The mitral valve has an anterior leaflet with a slightly long valve leaflet and a posterior leaflet with a short valve leaflet. They cause flow detachment when the inflow blood flow passes through the mitral valve during the diastole, resulting in a larger vortex around the anterior leaflet than that round the posterior leaflet. Three-dimensionally, a torus-shaped vortex is generated, called a vortex ring. The vortex at the posterior leaflet of the mitral valve disappears early in diastole, but the vortex around the anterior leaflet slightly moves to the apex and disappears during mid diastole. During the atrial systole, a vortex flow again develops around the anterior leaflet of the mitral valve, and a vortex that occupies a large portion of the left ventricle is generated around the base of the left ventricle toward the isovolumetric contraction. Simultaneously with the opening of the aortic valve, blood flow ejects as if blood flow is caused from this vortex. It is known that in the mid systole, the vortex disappears in the left ventricle with good cardiac function, but the vortex remains until the late systole in the left ventricle with deteriorated cardiac function.

As described above, the source of the left ventricular blood flow is the contraction and relaxation of the myocardium. That is, blood flows out and flows in from the left ventricle by opening and closing the aortic valve and the mitral valve. Further, in the heart, there are no physical boundaries in the blood flow path in the left ventricular cavity, except in special situation when there are intraventricular floating thrombi, floating tumors, and floating debris. In other words, topologically, it can be regarded identically as a three-dimensional open sphere and that there are no physical boundaries inside.

Hereinafter, in order to discuss the characteristic vortex flow of the left ventricle, the left ventricular long axis cross-sectional plane will be described in focus. In the left ventricle, the cross-sectional plane connects the central point of the aortic valve, that of the mitral valve, and the apex is called the apical long axis plane. It has an almost plane-symmetrical structure in healthy subjects, except for diseases such as ventricular aneurysm and myocardial infarction that accompany local degenerate in the myocardial wall. In the long axis plane, both the inflow and outflow blood flow velocities have the highest value. Therefore, the blood flow vector drawn in this plane should be a projection of the three-dimensional blood flow vector onto the two-dimensional cross-sectional plane, but the component of the in-plane vector is assumed to be larger than the component passing through the intersect section. As for the for the vortex flow in the left ventricular blood flow, the flow vector and trajectory in this cross-sectional plane are assumed to capture the characteristics of the three-dimensional left ventricular flow well.

The present description does not impose the two-dimensional blood flow in this long axis cross-sectional plane. However, as will be described later, the present description incorporates source and sink, and the local two-dimensional breakdown is consistent with the theory (previous research to be described later). The boundary closure in this cross-sectional plane is characterized by the presence of the left ventricular myocardium on the posterior and septal sides with the apex as the blind end, forming a boundary curve with the aortic valve on the septal side as the outlet passing through the center of the right coronary apex and commissure the left coronary cusp and non-coronary cusp, and with the mitral valve on the lateral side as the inlet passing through linear symmetrical plane of middle of the anteroposterior leaflet. On these boundaries, the mitral valve is a source boundary during diastole and the aortic valve is a sink boundary during systole, unless valve regurgitation is present. In the presence of valve regurgitation, the theory is basically unchanged because the structure will allow source and sink in the part of the anatomical structures. In addition, the myocardium does not move uniformly, and there are parts that contract early and those that contract slightly later. Therefore, it becomes a boundary where the parts that become source and sink are mixed in each phase. On the other hand, in the intraventricular region, it may be considered that there are no physical boundaries in the ventricular cavity in this cross-sectional plane, and there is no problem even if it is topologically identical to the open disk.

Hereafter, the multiple structures (vortices, source, sink, etc.) or vector fields that constitute a flow of finite type (hereinafter simply written as "flow") pattern is referred to as a "streamline structure" or simply a "structure". From the results of diligent studies, the present inventors have found that the streamline structures generated by the flow of finite type on a surface S (i.e., a streamline structure constituting an arbitrary flow pattern in a two-dimensional domain) and the corresponding characters, i.e., a "partial circle ordered rooted tree representation". Hereafter, a flow pattern expressed by combining these character strings is referred to as a "word representation". Hereafter, this finding will be referred to as the "prior research ". In the present description, the "partial circle order rooted tree representation" is referred to as a "COT representation". (COT: partially Cyclically Ordered rooted Tree representation). These streamline structures and their corresponding COT representations will be described below.

Hereinafter, the set of singular points of the flow v is denoted by Sing (v), the set of periodic orbits is denoted by Per (v), and the set of non-closed orbits is denoted by P (v).

First, the definitions are provided below.

(Definition 1) "The set Bd (v) of border orbits of the flow of finite type v on the surface S is given below.

$$\mathrm{Bd\,(v):= Sing\,(v)\ U\partial Per\,(v)\ \ U\partial P\,(v)\ \ UP\,sep\,(v)\ \ U\partial\,Per\,(v)}$$

Here, each set is an orbit set given below.

(1) P sep (v): the union of saddle separatrices and ss-separatrices in P(v),
(2) ∂Per (v): the boundary of the union of periodic orbits,
(3) ∂P (v): the boundary of the union of non-closed orbits, and
(4) $\partial_{per}$ (v): the union of periodic orbits along boundaries ∂S of S in ∂S∩intPer (v), and (Bd (v))$^c$ is defined by (Bd (v))$^c$ = S-Bd (v ).

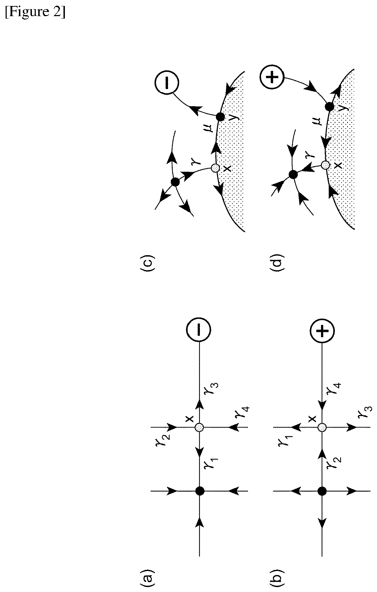

FIG. 2 (a) and 2 (b) show the point x of the slidable saddle. The set of a saddle point x, a ss-separatrix connected to it, and a source structure (sink structure) is called a "slidable saddle structure". The saddle point x is connected to four separatrices. When these are denoted by $\gamma_1$, $\gamma_2$, $\gamma_3$, and $\gamma_4$, $\alpha(\gamma_1) = \alpha(\gamma_3) = \omega(\gamma_2) = \omega(\gamma_4) = x$ is satisfied. The "saddle point x is slidable" means either the case in which "$\omega$ ($\gamma_1$) is a sink and $\omega$ ($\gamma_3$) is a sink structure (represented by a symbol - enclosed by ∘ in FIG. **2** (a)" or the case in which "$\alpha$ ($\gamma_2$) is the source and $\alpha$ ($\gamma_4$) is the source structure (represented by a symbol + enclosed by ∘ in FIG. **2**(b)".

FIG. **2**(c) and **2**(d) show the point x of the slidable $\partial$-saddle. "The boundary saddle x is slidable (or x is the slidable $\partial$-saddle)" refers to a structure in which there is a separatrix $\gamma \subset$ int S, a separatrix $\mu$ along the boundary, and a saddle y $\neq$ x on the same boundary as x on $\mu$, where y is connected to the sink structure, when $\omega$ ($\gamma$) = x, $\alpha$ ($\gamma$) has a source point, and $\alpha$ ($\mu$) = x, $\omega$ ($\mu$) = y (FIG. **2**(c)). A structure in which the directions of these vectors are reversed is also a slidable $\partial$-saddle (FIG. 2(d)).

In the word representation / tree representation theory of the topological orbit structure of the flow of finite type in the previous studies, an adjacency relationship of domains divided by this border orbit (mathematically represented as (Bd (v))$^c$ = S-Bd (v) has been represented as a graph, and a discrete combination structure such as a character string or a tree has been assigned thereto. Also in the present description, a character is assigned to a group of orbits contained in a two-dimensional domain divided into border orbits such that ( Bdh (v))$^c$ = S-Bd$^h$ ( v). Therefore, the concept of an orbit space obtained by introducing some kind of equivalence relationship into the orbit group is necessary.

(Definition 2) "A proper orbit group generated by the flow v on a surface S passes through the inside of an open subset T (S). An orbit space (orbit space) T/~ of T is a quotient set introduced from a following equivalence relationship "if arbitrary x, y $\in$ T, and O(x) = O(y), then x~y".

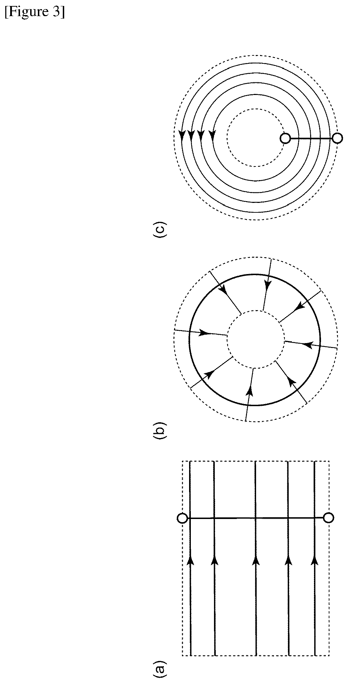

This quotient set means an operation of collapsing points on the same orbit into one point and identifying them. For example, in a case where uniform flows are parallel in an open box T as illustrated in FIG. **3**(a), the orbit space is the open interval. Similarly, as illustrated in FIG. **3**(b) and **3**(c), the orbit space of the orbit group in the open annulus comprises a circle and an open interval, respectively.

FIG. **3** illustrates a classification of orbit groups in a domain appearing in a complementary set of the set Bd(v) of border orbits of the flow of finite type: v. FIG. **3**(a) illustrates an open box where the orbit space is an open interval. That is, in a case where uniform flows are parallel in an open box T as illustrated in FIG. **3**(a), the orbit space is the open interval. FIG. **3**(b) illustrates an open annulus where the orbit space is a circle. FIG. **3**(c) illustrates an open annulus where an orbit space is an open interval. As illustrated in FIG. **3**, the orbit space of the orbit group in the open annulus comprises a circle and an open interval, respectively.

[Two-dimensional structure]

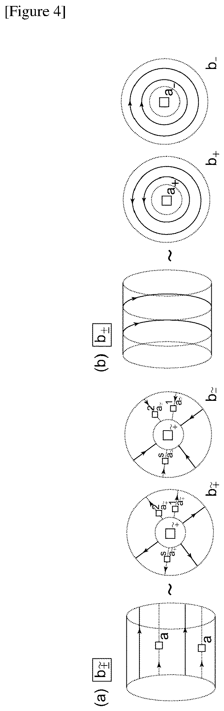

First, this two-dimensional domain structure is defined, and then the characters (COT representation) corresponding thereto are given. It is proven that there are only three types illustrated in FIG. **3** of the orbit groups that fill the open domains divided by Bd(v)". The open box illustrated in FIG. **3**(a) includes a non-closed orbit group in the neighborhood of the ss-separatrix connecting the source structure and the sink structure. Here, the conversion algorithm explained below does not assign a symbol to this non-closed orbit group. That is, this is a default structure. The characters (COT representation) are assigned to two two-dimensional structures other than this. FIG. **4** illustrates a structure (two-dimensional structure) representing the orbit group shown in FIG. **3**(b) and **3**(c).

(Two-dimensional structure: $b_{\sim\pm}$)

The structure of the open annulus illustrated in FIG. **3**(b) is in a situation filled with the non-closed orbits from the outside to the inside. For this, a symbol $b_{\sim\pm}$ is assigned as in FIG. **4**(a). For signs $\sim \pm$ attached to the symbol, when the orbit group flows from the outer boundary to the inner boundary of an annulus, this is denoted by $\sim$- for representing that the flow is sucked into the center. On the other hand, in a case where the orbit group spreads from the inner boundary to the outer boundary, this is denoted by $\sim$+. The sink/source structure always enters the inner boundary, and such set of Bd(v) structures is denoted by $\square_{\sim\pm}$. Since an arbitrary number (s $\geq$ 0) of class-$a_{\sim\pm}$ orbit structures of Bd(v) denoted by $\square_{a\sim\pm}$ may be put in each of the non-closed orbits filling the domain, a symbol representing the same is set to $\square_{a\sim\pm s}$. An order of structures corresponding to $\square_{a\sim\pm s}$ is not uniquely determined in a circular order. On the basis of the above-described discussion, the COT representation is $b_{\sim\pm}$ ($\square_{\sim\pm}$, $\{\square_{a\sim\pm s}\}$) (double-sign in the same order). Further, $\square_{a\sim\pm s}$ is the same as the notation in the case of $\square_{as}$ incompressible flow as

$$\square_{a\sim\pm s} := \square^1_{a\sim\pm} \cdots \square^s_{a\sim\pm} \; (s > 0)$$

$$\square_{a\sim\pm s} := \lambda_\sim \ (s = 0)$$

and the symbol $\lambda_\sim$ indicates that "nothing is contained".

(Two-dimensional structure: $b_\pm$)

FIG. **3**(c) illustrates a situation when an open disk is filled with the periodic orbits. The streamline structure corresponding to this is a structure $b_\pm$ illustrated in FIG. **4**(b). A sign + is assigned when the periodic orbits rotate counterclockwise (in a positive direction) and a sign - is assigned when they rotate clockwise (in a negative direction). The structure inside these structures is also an element of Bd(v), but since it is a class-a orbit structure that enters there, this is denoted by $\square_{\alpha\pm}$. Unlike the non-closed orbit, the structure such as $\square_\alpha$ does not enter in the periodic orbit, so this COT representation is given as b$\pm$ ($\square_{\alpha\pm}$) with double-sing in the same order.

Hereinafter, the structure of the orbit group that might be included in the domain divided by Bd(v) is selected from above. Herein, for use in the COT representation defined for Bd(v), sets defined from the structure of (Bd(v))$^c$ , $\square_{b\phi}$, $\square_{b+}$, $\square_{b-}$, $\square_{b\sim+}$, $\square_{b\sim-}$ are defined as follows.

$$\square_{b+} = \{b_{\sim\pm}, b_+\}$$

$$\square_{b-} = \{b_{\sim\pm}, b_-\}$$

$$\square_{b\sim+} = \{b_{\sim+}\}$$

$$\square_{b\sim-} = \{b_{\sim-}\}$$

[Root structure]

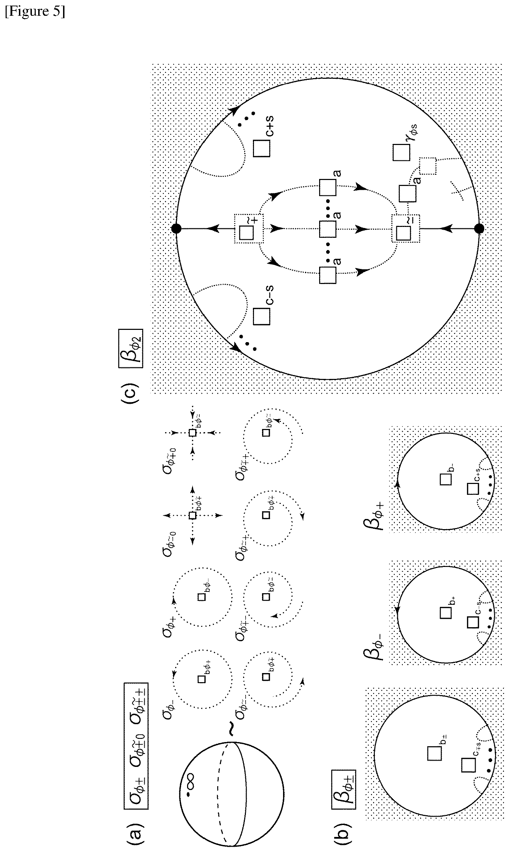

A plane can be topologically identified with a spherical surface S if the point of infinity is removed. The following basic flows are present on the spherical surface S. Hereinafter, these flow structures are referred to as a "root structure" indicating fundamental structure of the flow. FIG. 5 illustrates the root structure on a spherical surface S.

(Root structure: $\sigma\phi_\pm$, $\sigma\phi_{\pm 0}$ , $\sigma\phi_{\sim\pm\pm}$, $\sigma\phi_{\sim\pm\mp}$)

The flow field in a plane without physical boundaries can be identified with the flow on the spherical surface as illustrated in FIG. 5(a). This flow of finite type on the spherical surface gives flow in the annulus without two zero-dimensional point structures on both poles. In this situation, the classification of the COT representation of this structure depends on the orbit structure contained inside the flow in the annulus. When this annulus is filled with an orbit group structure $b_\pm$ formed of the periodic orbits given by the structure b$\pm$, a representation $\sigma_{\phi\mp}(\square_{b\phi\pm})$ is assigned. Here, $\square_{b\phi\pm}$ = $b_\pm(\square_{\alpha\pm})$. On the other hand, when the annulus is filled with non-closed orbit group structure $b_\pm$ of source/sink belonging to the class-$\sim\pm$, the COT representation is any one of $\sigma_{\phi\mp0}(\square_{b\phi\sim\pm})$ , $\sigma_{\phi\mp\pm}(\square_{b\phi\sim\pm})$ , and $\sigma_{\phi\mp\mp}(\square_{b\phi\sim\pm})$ depending on the rotational direction of the orbit around the source/sink. That is, around the source/sink at infinity, when the non-closed orbit group does not rotate, $\sigma_{\phi\sim\mp+}(\square_{b\phi\sim\pm})$ in double-sign in same order, when this rotates counterclockwise, $\sigma_{\phi\sim\mp+}(\square_{b\phi\sim\pm})$ in double-sign in same order, and when this rotates clockwise, $\sigma_{\phi\sim\mp-}(\square_{b\phi\sim\pm})$ in double-sign in same order. Here, $\square_{b\phi\sim\pm} = b_{\sim\pm}(\square_{\sim\pm}, \{\square_{a\sim\pm s}\})$. Note that the signs assigned to the symbols of $\sigma_{\phi\sim\pm0}$, $\sigma_{\phi\sim\mp\mp}$, and $\sigma_{\phi\sim\mp\pm}$ are opposite to the signs of the COT representation of a two-dimensional orbit group structure contained there because the sign is assigned to the structure of the flow around the point corresponding to the point at infinity. For example, for the periodic orbit in the counterclockwise direction (that is, + direction) with the representation of b+ inside, a flow in a clockwise direction (that is, - direction) must occur around the point at infinity. Therefore, specific COT representation is

$$\sigma_{\phi-}(\square_{b\phi+})$$

$$\square_{b\phi+} = b_+(\square_{\alpha+})$$

(Root structure: $\beta_{\phi\pm}$, $\beta_{\phi2}$)

Suppose that a spherical surface includes some physical boundaries. In this situation, it is possible to select one of them as a special boundary, and introduce spherical polar coordinates such that a north pole is included in the boundary. In this situation, the flow on the spherical surface can be identified with an inner flow in the two-dimensional bounded domain through a stereographic projection associated with this coordinate system. FIG 5(b) illustrates a flow that is a child of the root and includes no source/sink structure on the outer physical boundaries. The COT representation is given as $\beta_{\phi-}$ ($\square_{b+}$, $\{\square_{c\text{-s}}\}$) when the flow on the outer boundary is counterclockwise. Although this structure must constantly include a class-b+ structure in $\square_{b+}$, it is possible to attach an arbitrary number of class-c-orbit structures on the outer boundary. When the flow direction on the outer boundary is clockwise, all the signs are inverted to obtain the root structure having the COT representation of $\beta_{\phi+}(\square_{b-}, \{\square_{c+s}\})$. Note that, in both cases, $\square_{c\pm s}$ that means $s \geqq 0$ class-c orbit structures can be specifically represented as follows in double-sign in same order.

[Equation 1]

$$\square_{c\pm s} := \underbrace{\square_{c\pm}^{1} \cdots \cdots \square_{c\pm}^{S}}_{s} \quad (s > 0), \qquad \square_{c\pm s} := \lambda_{\pm} \quad (s = 0).$$

FIG. 5(c) illustrates a basic flow structure in which there is at least one source/sink structure connected to the outer physical boundary. A root structure $\beta_{\phi2}$ is the basic flow structure illustrated in FIG. 5(c). A pair on a leftmost side is selected from the source/sink structures as a class-$\sim\pm$ special orbit structure, and a class-$\gamma_{\phi}$ orbit structure is assigned to other source/sink structures. In addition, an arbitrary number of class-c$\pm$ orbit structures may be attached to right and left along the boundary. This situation is represented in the COT representation by $\square_{\sim\pm}$, for the special pair of source-sink and by $\square_{\gamma\phi s}$ for an arbitrary number of class-$\gamma_{\phi s}$ orbit structures. Specifically, this may be represented as follows.

[Equation 2]

$$\square_{\gamma\phi s} := \underbrace{\square_{\gamma\phi}^{1} \cdots \cdots \square_{\gamma\phi}^{S}}_{s} \quad (s > 0), \qquad \square_{\gamma\phi s} := \lambda_{\sim} \quad (s = 0).$$

Since there is the special pair of source-sink, an arbitrary number of class-a orbit structures connected to them can be attached. They are represented as $\square_{as}$ in COT representation. Refer to Table 1 for the definition of the class-a structure group that may include $\square_{as}$. As a similar manner to the notation in an incompressible flow, $\square_{as}$ is determined as:

$$\square_{as} := \square_{a}^{1} \cdots \square_{a}^{s} \ (s > 0)$$

$$\square_{as} := \lambda_{\sim} \ (s = 0)$$

In summary, COT representation of this root structure is given as

$$\beta_{\phi2}(\{\square_{c+s}, \square_{\sim+}, \square_{c\text{-s}}, \square_{\sim\sim}, \square_{\gamma\phi s}\}, \square_{as})$$

by allocating each structure attached to the outer boundary counterclockwise in a cyclical order.

[Zero-Dimensional Point Structure and One-Dimensional Flow Structure in Bd(v)]

Next, classification of the zero-dimensional point structure and the one-dimensional flow structure in Bd(v) forming the ss-saddle connection diagram Dss(v) defined by the flow of finite type v on a surface S, and corresponding COT representation are given. According to the above-described theory, since it is represented as Bd(v) = Sing(v) U

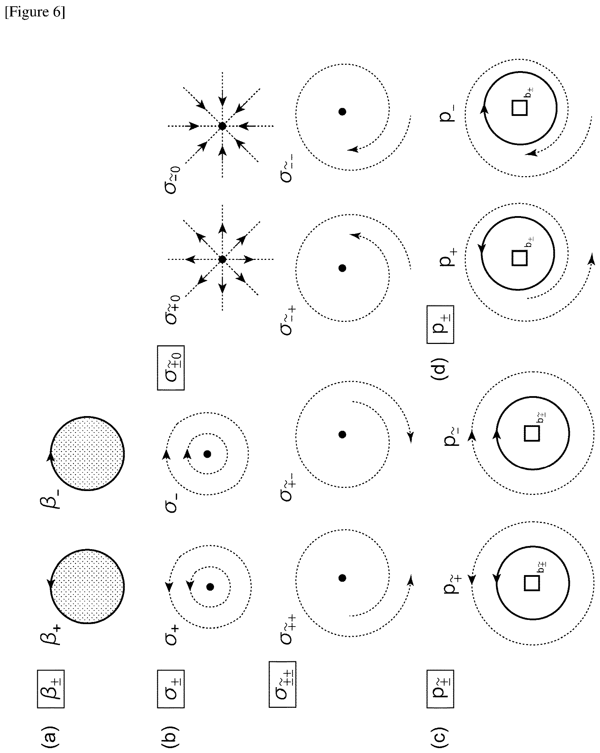

$\partial$Per(v) U $\partial$P(v) U P$_{sep}$ (v) U $\partial_{per}$(v), the zero-dimensional point structure and the one-dimensional structure that realize Bd(v) are introduced corresponding to each set. Note that a set where each one-dimensional structure enter could depend on orbit group information around the it. FIG. 6 illustrates samples of the zero-dimensional point structure and the one-dimensional structure in Bd(v).

[Structure of $\partial_{per}$(v), Sing(v)]

(One-dimensional structure: $\beta_{\pm}$ )

By definition, the flow of $\partial_{per}$(v) refers to the periodic orbit flowing along the physical boundaries. A symbol $\beta$ represents flow structure whose physical boundaries are not connected to any class-c structure enclosed by the $\partial$-saddle separatrix. That is, COT representation is given as $\beta_+\{\lambda_+\}$ when the flow on the physical boundary is counterclockwise, and as $\beta$-{$\lambda$-} when the flow is clockwise (refer to FIG. **6**(a)). These physical boundaries belong to class- $\sim\pm$ and class-$\alpha_+$ structures.

(Zero-Dimensional Point Structure: $\sigma_{\pm}$, $\sigma_{\sim\pm0}$, $\sigma_{\sim\pm\pm}$, and $\sigma_{\sim\pm\mp}$)

An element of Sing(v)\Dss(v) is the zero-dimensional point structure (isolated structure). The point structures can be classified by the orbit around it. When the point is a center accompanied by counterclockwise or clockwise periodic orbits around it, the COT representation is given by $\sigma_+$ and $\sigma_-$, respectively. On the other hand, when the point is a source or the sink, the COT representation is given by $\sigma_{\sim\pm0}$, $\sigma_{\sim\pm\pm}$, and $\sigma_{\sim\pm\mp}$ along with the rotational direction of the orbit around it (refer to FIG. 6(b)). These point structures belong to class--$\pm$ structure.

[Structures belonging to $\partial$P(v) and $\partial$Per(v)]

(One-Dimensional Structure: p$_{\sim\pm}$, p$_{\pm}$)

The sets $\partial$P(v) and $\partial$Per(v) are the one-dimensional structures defined as boundary sets of non-closed orbits and periodic orbits, respectively. The limit cycle is the periodic orbit in which either its inner side or outer side is a limit orbit of a non-closed orbit. Since this is not an element of intP(v), this is not a structure that can be an element of a set, Psep(v). In this situation, classification is required depending on structures on the outer side and the inner side of the limit cycle. That is, one is a structure of the periodic orbit in an outer domain of the limit cycle illustrated in FIG. 6(c), and the other is a structure in which the limit cycle illustrated in FIG. 6(d) is the $\omega(\alpha)$-limit set of the non-closed orbits in the outer domain. The former structure is denoted by p$_{\sim\pm}$. In order for this periodic orbit to be the limit cycle in this situation, this must be the limit orbit of the non-closed orbit from the inside (that is, the border orbit). Therefore, the two-dimensional structure of b$_{\sim\pm}$ is put inside it. Therefore, the COT representation is p$_{\sim\pm}$ ($\square_{b\sim\pm}$). The latter structure is represented by a symbol p$_{\pm}$. In this situation, since this is a limit periodic orbit from the outside, the structure of an inner two-dimensional limit orbit group can be any structure. Therefore, the COT representation thereof may be p$_{\pm}$ ($\square_{b\pm}$) in double-sign in same order.

[Structures belonging to $\partial$P(v), $\partial$Per(v), and P$_{sep}$(v)]

The one-dimensional structure that can be any of structure sets of $\partial$P(v), $\partial$Per(v), and P$_{sep}$(v) has a non-closed orbit including the structure of the saddle separatrix or ss-separatrix. Two-dimensional structures inside its inner and/or outer part can either be a domain where one is filled with the non-closed orbits and the other is filled with the periodic orbits (in this situation, it should be $\partial$P(v) or $\partial$Per(v)), or a domain where both the sides are filled with the non-closed orbits (in this situation, it should be the element of P$_{sep}$(v)).
First, since there are four separatrices connected to a saddle, there are three following possibilities considering local flow directions of the separatrices.

(S1) One is connected to the source structure, one is connected to the sink structure, and the other two are self-connected saddle separatrices.
(S2) There are two self-connected saddle connections.
(S3) The two are connected to the source (sink) structures. Note that the other two cannot be the self-connected separatrices from the direction of flow due to topological constraints.

Among them, saddle separatrices in (S3) pattern do not have non-closed orbit, so that only (S1) and (S2) should be considered. On the other hand, there are three separatrices from a boundary saddle, but since two of them need to be on the boundary, there is only one degree of freedom. Therefore, there are following two possibilities of the connected structure.

(S4) This has the $\partial$-saddle separatrix connected to another $\partial$-saddle on the same boundary.
(S5) This is connected to the source/sink structure inside the domain.

In a case of (S4), there is no problem because non-closed orbit is obviously formed, but a case of (S5) depends on a surrounding situation. That is, in this case, the non-closed orbit does not occur by itself. However, in relation to the index of the singular points of the vector field, both one boundary saddle point and at least another one boundary saddle point should exist. Therefore, when the boundary saddle point has the $\partial$-saddle separatrix, an entire structure might include the non-closed orbit. From above, four structures corresponding to (S1), (S2), (S4), and (S5) will be considered below.

(One-dimensional structure: $a_\pm$, $q_\pm$)

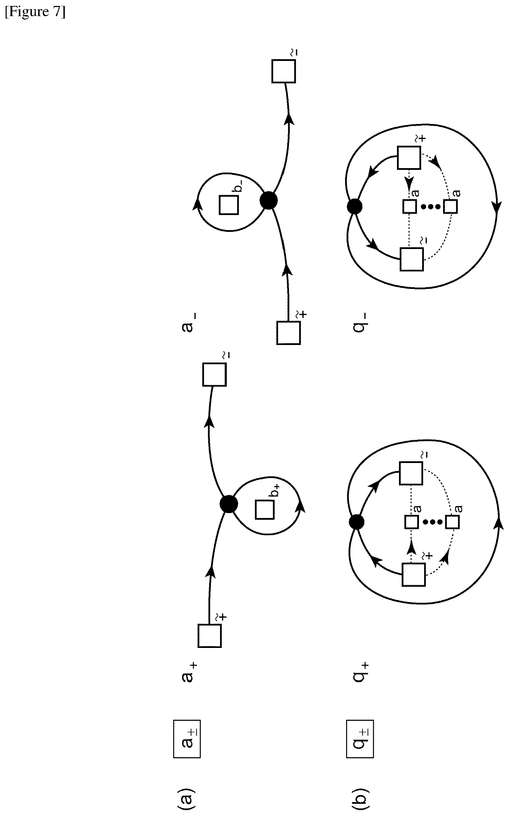

FIG. 7 illustrates one-dimensional structures in (S1)-series. These structures are classified according to the configurations among the source structure, sink structure, and self-connected saddle separatrix.
First, a structure illustrated in FIG. 7(a), that is, the structure in which the source / sink structure is present outside surrounding the self-connected saddle separatrix is denoted by $a_\pm$. A sign is determined to be $a_+$ in a case when the self-connected saddle separatrix is counterclockwise and $a_-$ in a case when it is clockwise. In this structure, the periodic orbit or non-closed orbit might enter the self-connected saddle separatrix. In a case where the periodic orbit locates inside the self-connected saddle, the rotational direction inside it is automatically determined, so that a structure set defined by $\square_{b+}$ substitutes a+, and a structure set defined by $\square_{b-}$ substitutes a_.
Next, a structure illustrated in FIG. 7(b), that is, the structure in which the source / sink structures are located inside the domain surrounded by the self-connected saddle separatrix is denoted by $q_\pm$. A sign is $q_+$ when the direction of the outer self-connected saddle separatrix is counterclockwise, and $q_-$ when the direction is clockwise. Inside this structure, there can be source/sink structures surrounded by this structure and an arbitrary number ($s \geqq 0$) of elements of the structure set connecting to them, so that the COT representation is $q_\pm$ ($\square_{\sim+}$, $\square_{\sim-}$, $\square_{as}$).

(One-dimensional structure: $b_{\pm\pm}$, $b_{\pm\mp}$)

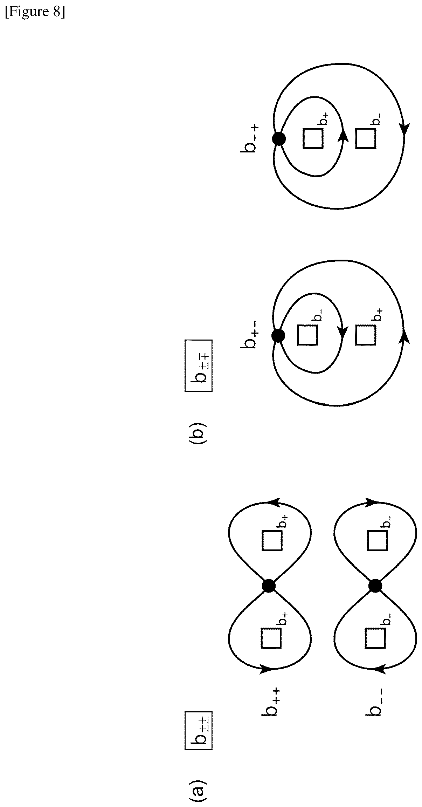

FIG. 8 illustrates (S2)-series one-dimensional structures. Here, the structures are classified according to the configurations among the two self-connected saddle separatrices.
First, a structure illustrated in FIG. 8(a), that is, the structure in which domains surrounded by two self-connected saddle separatrices are located on the outer sides of each other is denoted by $b_{\pm\pm}$ in double-sign in same order. A sign is $b_{++}$ when the rotational directions of the two self-connected saddle separatrices are counterclockwise, and $b_{--}$ when these directions are clockwise. The two-dimensional structures can locate inside the domains surrounded by these two self-connected saddle separatrices. In a case where the periodic orbits locates inside the domain, the rotational direction is automatically determined, and since the order of these two domains is arbitral, so that they are enclosed by {}, and the COT representations thereof are $b_{++}$ {$\square_{b+}$, $\square_{b+}$} and $b_{--}$ {$\square_{b-}$, $\square_{b-}$}.
Next, a structure illustrated in FIG. 8(b), that is, the structure in which, a domain surrounded by one self-connected saddle separatrix has another self-connected saddle separatrix inside is included is denoted by $b_{\pm\mp}$ in double-sign in same order. A sign is assigned to be $b_{+-}$ when the direction of the outer self-connected saddle separatrix is counterclockwise, and $b_{-+}$ when the direction is clockwise. From the method of determining the sign, in a case where the orbit group inside is the periodic, it is automatically determined that the rotational directions are opposite to each other. Therefore, the COT representations are $b_{+-}(\square_{b+}, \square_{b-})$ and $b_{-+}(\square_{b-}, \square_{b+})$. Here, the signs under b are determined corresponding to the order of the inner structures.

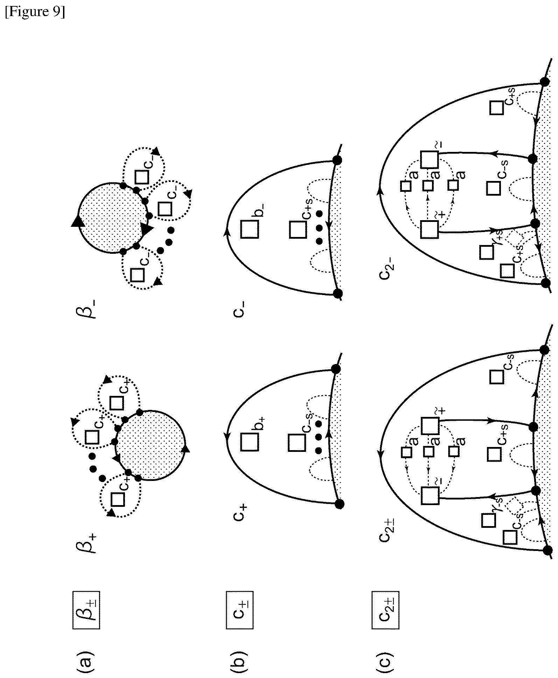

FIG. 9 illustrates one-dimensional structures in (S4)-series.

(One-dimensional structure: $\beta_\pm$)

A structure illustrated in FIG. 9(a) corresponds to the physical boundary where an arbitrary number of $\alpha$-saddle separatrices are attached. If no $\alpha$-saddle separatrix is attached, it is identical to a form illustrated in FIG. 6(a), and the COT representation is $\beta_\pm\{\lambda_\pm\}$. On the other hand, when one or more $\alpha$-saddle separatrices are attached to the boundary, the COT representation is given $\beta_+\{\square_{c+s}\}$ in a case where the flow is counterclockwise along the boundary, and $\beta_-\{\square_{c-s}\}$ is given in a case where the flow is clockwise (refer to FIG. 9(a)). That is, the following symbols are cyclically ordered with each structure counterclockwise in $\square_{c\pm s}$.

$$\Box_{c\pm s} := \Box^1{}_{c\pm} \cdots \Box^s{}_{c\pm} \ (s>0)$$

$$\Box_{c\pm s} := \lambda_\pm \cdots \ (s=0)$$

(One-dimensional structure: $c_\pm$, $c_{2\pm}$)

One-dimensional structures $c_+$ and $c_{2+}$ belong to the (S4)-series. As illustrated in FIG. **9**(b) and **9**(c), they can be classified according to the structure surrounded by the $\alpha$-saddle separatrix.

First, when the two-dimensional structure filled with the periodic orbits or non-closed orbits, that is, when there are no source /sink structures connected to the boundary saddle, a structure illustrated in FIG. **9**(b) is obtained. This is denoted by $c_+$. A sign is assigned to be + when a rotational direction is counterclockwise, and - when the direction is clockwise in the orbit along the $\partial$-saddle separatrix and the boundary. In this situation, when all the inner orbit group is periodic, the direction is automatically determined. Note that an arbitrary number of $c_+$ structures can be further included inside, but in this case the rotational direction of the inner domain would be opposite. On the basis of this, a set of $c_+$ structures is defined as:

$$\Box_{c+} = \{c_+\}$$

$$\Box_{c-} = \{c_-\}$$

When the structure set of $\Box_{c\pm s}$ is defined in double-sign in same order to the arbitrary number (s $\geqq$ 0) of them, the COT representation thereof is $c_\pm(\Box_{b\pm}, \Box_{c\mp s})$ in double-sign in same order.

Next, in a case where the source/sink structures are located inside, these structures should be connected to the saddle on the same boundary under the requirement of the index of the singular points of the vector field. That is, the structure illustrated in FIG. **9**(c), that is, the structure in which the slidable $\partial$-saddle is surrounded by the $\partial$-saddle separatrix is denoted by $C_{2\pm}$. In general, any number of slidable $\partial$-saddles, structures of the connection from a source / sink to the boundary, be attached to the boundary, so that a rightmost one is selected, and the corresponding pair of source / sink structures is represented as $\Box_{\sim\pm}$. The structure set $\Box_{as}$ is included indicating that there are an arbitrary number (s $\geqq$ 0) of structure sets $\Box_a$ connecting the source/sink structures. Furthermore, on the boundary, in addition to an arbitrary number (s $\geqq$ 0) of $c_+$ structures depending on their directions, there can be a set $\Box_{\gamma\mp s}$ representing an arbitrary number of (s $\geqq$ 0) of slidable $\partial$-saddle structures. Note that the reason that the structure of $\Box_{\gamma\pm s}$ is always on the left side is that the rightmost one of a total of (s + 1) slidable $\partial$-saddle structures is selected and represented as $\Box_{\sim\pm}$. Here, in order to give the COT representation of this structure, one rule is determined for the order of the inner structures. That is, "in a case where there is a structure surrounded by the $\partial$-saddle separatrix on a circular boundary inside, the inner structures are ordered in a counterclockwise direction as seen from the inside of the boundary. On the other hand, in a case of connecting to the outer boundary of $\beta_{\phi 2}$ above, the structures are ordered in a clockwise direction as seen from the inside of the boundary (conversely, in the counterclockwise direction as seen from a portion where a fluid is present)". According to this rule, the COT representation of the structure illustrated in FIG. 9(b) can be obtained by ordering the structures from a rightmost side as $c_{2+}(\Box_{c\mp s}, \Box_{\sim\pm}, \Box_{c\pm s}, \Box_{\sim\mp}, \Box_{\gamma\mp s}, \Box_{c\mp s}, \Box_{as})$ considering that the structure is attached to the inner boundary. Note that, when the rules are determined in this manner, the structure is such that the structures in the clockwise direction are always arranged regardless of the inner and outer boundaries.

(One-dimensional structure: $a_2$, $\gamma_{\phi\sim\pm}$, $\gamma_{\sim\pm\pm}$)

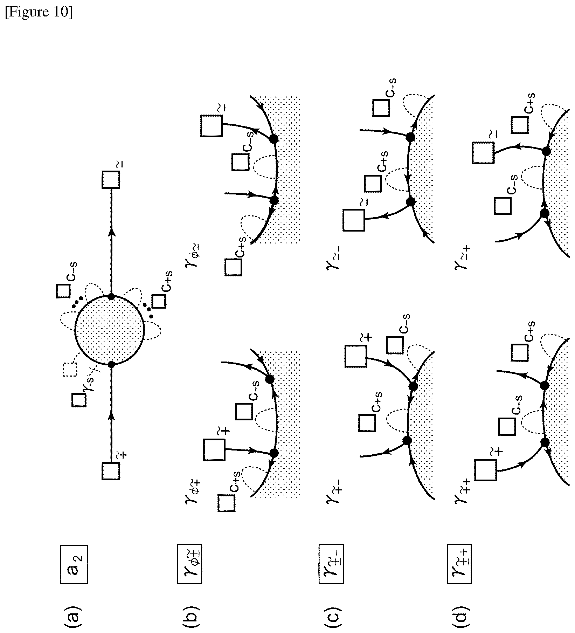

FIG. 10 illustrates one-dimensional structures in (S5)-series. This one-dimensional structure basically corresponds to the slidable $\partial$-saddle connecting a pair of source / sink structures on the boundary. For convenience of a later algorithm configuration, in a case where there is a plurality of such structures, one of them is treated as a special structure.

The structure illustrated in FIG. **10**(a) is the structure representing a special one of the slidable saddles connecting the pair of source/sink structures, and is denoted by $a_2$. Any arbitrary number (s $\geqq$ 0) of structures $\Box_{c\pm}$ surrounded by the $\partial$-saddle separatrix can be attached to the physical boundary depending on a flow direction on the boundary. This is denoted by $\Box_{c\pm s}$. Any arbitrary number of other slidable $\partial$-saddle structures can be attached, but when a lowermost slidable $\partial$-saddle is selected specifically, there are s $\geqq$ 0 structures $\Box_{\gamma-}$ of other slidable $\partial$-saddle only on an upper side thereof. This is represented as $\gamma_{\sim-+}$ Finally, the COT representation can be made $a_2(\Box_{c+s}, \Box_{c-s}, \Box_{\gamma-s})$ by ordering the structures counterclockwise on the boundary according to a rule of order of the structures of the COT representation with respect to the structure attached to an inner circular boundary. Note that the reason that $\gamma_{\sim-+}$ locates only on a left side of $\square_{c\text{-}s}$ is by the definition of a structure $\gamma_{\sim\pm\pm}$ to be introduced later.

After selecting the special slidable $\partial$-saddle connecting the source/sink structures, all other slidable $\alpha$-saddles need to be treated equally. Structures added here must be classified by the structure of the boundary to which they are attached. First, an arbitrary number of structures may be attached to an outer circular boundary of $\beta_{\phi2}$, but in the definition of $\beta_{\varphi2}$, a symbol of $\square_{\sim\pm}$ is assigned to a pair structure of the source and sink is on the leftmost side always, so that all the others are on the right side. Since the flow proceeds from top to bottom along a right boundary, s $\geqq$ 0 slidable $\partial$-saddles may also be added in the same direction. At that time, two $\partial$-saddles are added in relation to the index of the singular points of the vector field. This makes it possible to sandwich the structure of $\square_{c\pm s}$ in between. The structure of $\square_{c+s}$ enters the right boundary along the flow. Therefore, in order to add the structure of slidable $\partial$-saddle beyond this, a structure as illustrated in FIG. **10**(b) is required. This structure is denoted by $\gamma_{\phi\sim\pm}$. The sign is $\gamma_{\phi\sim+}$ when a newly added structure is the source structure, and $\gamma_{\phi\sim-}$ when this is the sink structure. The COT representations of the respective structures are $\gamma_{\phi\sim+}$ ($\square_{c+s}$, $\square_{\sim+}$, $\square_{c\text{-}s}$) and $\gamma_{\phi\sim-}$ ($\square_{c+s}$, $\square_{c\text{-}s}$, $\square_{\sim-}$) because the structure is read counterclockwise (clockwise as seen from the outside inside) from left to right in a case of the structure attached to the outer boundary. Note that the existing sink (source) structure is connected to the $\alpha$-saddle point different from the $\partial$-saddle point connected to the added source (sink) structure.

Finally, the structure of the slidable $\partial$-saddle attached to the boundary at $a_2$ or $c_2$ includes two types of a structure added from the downstream side of the flow along the boundary (FIG. **10**(c)) and a structure added from the upstream side (FIG. **10**(d)). They are denoted by $\gamma_{\sim\pm-}$ and $\gamma_{-\pm+}$, respectively. Then, the sign is determined depending on whether a newly added structure is the source or the sink structure, respectively. The corresponding COT representations are $\gamma_{\sim+-}$ ($\square_{c\text{-}s}$, $\square_{\sim+}$, $\square_{c+s}$), $\gamma_{\sim--}$($\square_{c\text{-}s}$, $\square_{c+s}$, $\square_{\sim-}$), $\gamma_{\sim++}$($\square_{c+s}$, $\square_{c\text{-}s}$, $\square_{\sim+}$), and $\gamma_{\sim-+}$($\square_{c+s}$, $\square_{\sim-}$, $\square_{c\text{-}s}$) because the structure is located counterclockwise on the inner boundary. Note that there are s $\geqq$ 0 structures of $\gamma_{\sim\pm+}$, and s $\geqq$ 0 structures of $\gamma_{\sim\pm-}$ in herein introduced $\square_{\gamma+s}$ and $\square_{\gamma\text{-}s}$, respectively.

[Structures belonging to $P_{sep}(v)$]

(One-Dimensional Structure: $a_{\sim\pm}$, $q_{\sim\pm}$)

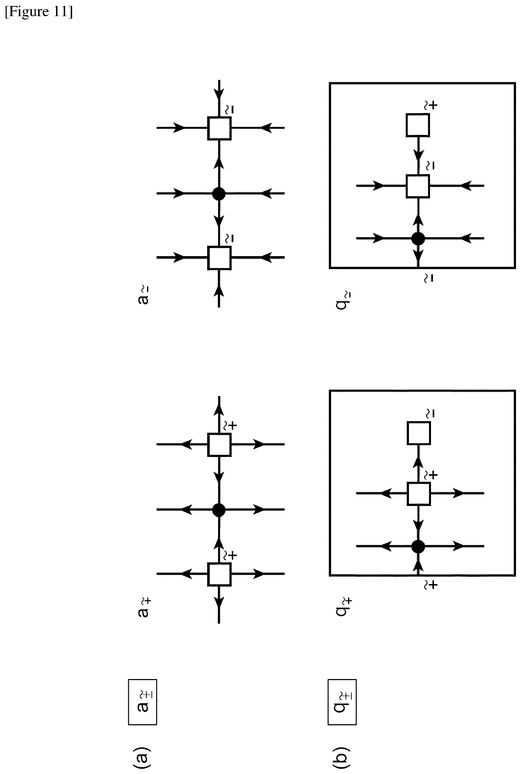

FIG. 11 illustrates one-dimensional structures in (S3)-series. They become slidable saddle structures since two source / sink structures are attached to the saddle point. The structures are classified according to the configuration of the ss-component connected to the slidable saddle. In this situation, since all the neighborhood orbits are two-dimensional domains (open box) filled with the non-closed orbits, they always become elements of Psep(v). A structure corresponding to the slidable saddle in FIG. **11**(a) present outside the ss-component to which the slidable saddle is connected is denoted by $a_{\sim\pm}$. A structure in FIG. **11** (b) present inside the ss-component to which the slidable saddle is connected corresponds to the slidable saddle. This is denoted by $q_{\sim\pm}$. A sign is assigned to be $a_{\sim+}$ in a case where a source structure $\square_{\sim+}$ is attached on bilateral sides, and $a_{\sim-}$ in a case where a sink structure $\square_{\sim-}$ is attached on bilateral sides. The order of these source/sink structures can be freely selected, so that the COT representations are $a_{\sim\pm}\{\square_{\sim\pm}, \square_{\sim\pm}\}$ and $q_{\sim\pm}(m_{\sim\pm})$ in double-sign in same order.

With regard to all the streamline structures described above, Table 1 illustrates a correspondence relationship between each of the streamline structures and their characters (COT representation).

[Table 1]

| Root structure | COT representation |
|---|---|
| $\sigma_{\emptyset\pm}$ | $\sigma_{\emptyset\mp}(\square_{b\emptyset\pm})$ |
| $\sigma_{\emptyset\pm0}$ | $\sigma_{\emptyset\pm0}(\square_{b\emptyset\widetilde{\mp}})$ |
| $\sigma_{\emptyset\widetilde{\pm}\pm}$ | $\sigma_{\emptyset\widetilde{\pm}+}(\square_{b\emptyset\widetilde{\mp}})$ |
| $\sigma_{\emptyset\widetilde{\pm}\mp}$ | $\sigma_{\emptyset\widetilde{\pm}-}(\square_{b\emptyset\widetilde{\mp}})$ |
| $\beta_{\emptyset\perp}$ | $\beta_{\emptyset\mp}(\square_{b\perp},\{\square_{c\mp s}\})$ |
| $\beta_{\emptyset2}$ | $\beta_{\emptyset2}(\{\square_{c+s},\square_{\widetilde{\mp}},\square_{c-s},\square_{\widetilde{\simeq}},\square_{\gamma\emptyset s}\},\square_{\alpha s})$ |

(continued)

| Two-dimensional structure | COT $_{\text{representation}}$ |
|---|---|
| $b_+^{\sim}$ | $b_{\mp}(\square_{\mp}, \{\square_{\alpha \mp s}\})$ |
| $b_{\pm}$ | $b_{\pm}(\square_{\alpha \pm})$ |
| **Singular structure** | COT $_{\text{representation}}$ |
| $\sigma_{\pm}$ | $\sigma_{\pm}$ |
| $\sigma_{\pm 0}^{\sim}$ | $\sigma_{\pm 0}^{\sim}$ |
| $\sigma_{\pm =}^{\sim}$ | $\sigma_{\pm \pm}^{\sim}$ |
| **Cycles** | COT $_{\text{representation}}$ |
| $p_{\pm}^{\sim}$ | $p_{\mp}(\square_{b \mp})$ |
| $p_{\pm}$ | $p_{\pm}(\square_{b+})$ |
| **Circuits** | COT $_{\text{representation}}$ |
| $a_{\pm}$ | $a_{\pm}(\square_{b\pm})$ |
| $q_{+}$ | $q_{+}(\square_{\mp}^{\sim}, \square_{\simeq}, \square_{\alpha s})$ |
| $b_{\pm =}$ | $b_{\pm =}\{\square_{b=}, \square_{b\pm}\}$ |
| $b_{\pm}$ | $b_{\pm =}(\square_{b\pm}, \square_{b\mp})$ |
| $\beta_{\pm}$ | $\beta_{\pm}\{\square_{c\pm s}\}$ |
| $c_{\pm}$ | $c_{\pm}(\square_{b\perp}, \square_{c\mp s})$ |
| $c_{2\pm}$ | $c_{2\pm}(\square_{c\top s}, \square_{\mp}^{\sim}, \square_{c\pm s}, \square_{\mp}^{\sim}, \square_{\gamma \top s}, \square_{c\top s}, \square_{\alpha s})$ |
| $a_{2}$ | $a_{2}(\square_{c+s}, \square_{c-s}, \square_{\gamma -s})$ |
| $\gamma_{\emptyset +}^{\sim}$ | $\gamma_{\emptyset \mp}^{\sim}(\square_{c+s}, \square_{\mp}^{\sim}, \square_{c-s})$ |
| $\gamma_{\emptyset -}^{\sim}$ | $\gamma_{\emptyset \simeq}(\square_{c+s}, \square_{c-s}, \square_{\simeq})$ |
| $\gamma_{+-}^{\sim}$ | $\gamma_{\mp -}^{\sim}(\square_{c-s}, \square_{\mp}^{\sim}, \square_{c+s})$ |
| $\gamma_{--}^{\sim}$ | $\gamma_{\simeq -}^{\sim}(\square_{c-s}, \square_{c+s}, \square_{\simeq})$ |
| $\gamma_{++}^{\sim}$ | $\gamma_{\mp +}^{\sim}(\square_{c+s}, \square_{c-s}, \square_{\mp}^{\sim})$ |
| $\gamma_{-+}^{\sim}$ | $\gamma_{\simeq +}^{\sim}(\square_{c+s}, \square_{\simeq}, \square_{c-s})$ |
| **Slidable saddles** | COT $_{\text{representation}}$ |
| $a_{\pm}^{\sim}$ | $a_{\mp}^{\sim}\{\square_{\mp}^{\sim}, \square_{\simeq}^{\sim}\}$ |
| $q_{\pm}^{\sim}$ | $q_{\mp}^{\sim}(\square_{\mp}^{\sim})$ |

However, in the intraventricular blood flow, the contraction and relaxation of the ventricular wall becomes the source and/or sink of the flow from the moving boundary. Therefore, the slip boundary condition on the physical boundary presupposed above cannot be applied, and the orbit group becomes transversal to the boundary. As a result, the structures including the myocardial wall and the heart valve become source / sink. Therefore, there is a problem that a sufficient COT expression cannot be given merely by the above (prior art) root structure.

The region of interest (ROI) in the present description is a two-dimensional cross-sectional plane where data inside a ventricle are obtained from measurement devices for intraventricular flow such as echocardiography and MRI. The boundaries of this ventricular domain consist of a part that allows a fluid to flow in and out through a valve and a moving boundary that changes over time during cardiac cycle. In the present description, these two boundaries are regarded as one without distinction, and the intraventricular domain is topologically identified as the two-dimensional open disk Ω. Strictly speaking, even though the heart beats deform the boundary shape from moment to moment, topologically it is still a disk, thus this hypothesis is valid.

Given that the target flow is the blood flow inside a ventricle, a hypothesis that there is no physical boundary inside Ω can be valid. On the other hand, considering that the target flow is composed from the image data of the orbit group obtained from the measurement of the flow in the ventricle (hereinafter referred to as "intraventricular blood flow image data"), note that at the circumferential boundary ∂Ω of ROI, the flow does not meet the slip boundary condition. That is, most of the orbit groups obtained by streamline visualization are sources and sinks of the domain flow, and crosses the circular boundary in a transversal manner. In the intraventricular blood flow image data, extractions of all the orbits are not always guaranteed, due to the influence of noise and insufficient measurement accuracy. Furthermore, it is assumed that some orbits contacts at a certain point on the boundary. However, in such a case, it can be assumed that the orbit group can be made transversal at all points of the boundary ∂Ω by cutting a little area in the vicinity of the contact point to make a new boundary. Under this circumstance, the following equivalence relations are introduced at points on the boundary.

$$\forall x, x' \in \ \Omega^-, x \sim x' \Leftrightarrow \ x, x' \in \ \partial\Omega$$

Here, Ω⁻ represents the closure of the set Ω.

Considering Ω⁻ / ~ obtained by this equivalence relation, this intraventricular domain can be topologically identified with a spherical surface S by degenerating the boundary into one point. Further, by introducing an appropriate spherical polar coordinate, representative element of this domain can be the North Pole in the sphere (the point at infinity in the plane, hereinafter referred to as {∞}). Since all the orbit groups are transversal to the boundary, an infinite number of orbits go in and out at this infinity, and the infinity point becomes a degenerate singular point. On the other hand, at other points on the sphere x ∈ S \ {∞} (that is, points on Ω), it is natural to assume that the flow is non-degenerate (regular non-degenerate). Therefore, the flow handled in the topology classification of the orbit group of the intraventricular blood flow image data results in the flow on the spherical surface S having one degenerate singular point at the point of infinity. The classification of the topology of the orbit group on the surface and this transformation theory to the discrete combination structure described above did not allow the existence of such a degenerate singular point. Therefore, the mathematical treatment of the flow of this degenerate singular point plays an important role in the topology classification of the orbit group created by the intraventricular blood flow by the moving boundary.

[New definition in zero-dimensional singularity structure]

In the present description, it is assumed that all singularities are non-degenerate within the region of interest Ω. In this situation, there are the following four types of non-degenerate singular points that can exist at the inner point of Ω.

- Center (center vortex point)
- saddle (saddle point)
- source (source point)
- sink (sink point)

Here, since it is assumed in the present description that there is no physical boundary inside the region of interest Ω, non-degrading singular points on the boundary such as ∂-saddle (boundary saddle point), ∂-source (boundary source point), and ∂-sink (boundary sink point) do not appear.

In the present description, in order to deal with orbit group crossing ∂Ω in a transversal manner in the intraventricular blood flow image data based under above assumption, it is necessary to successfully introduce a singular degenerative point at infinity to develop topological classification. In addition, the existence of an infinitesimal orbit group from the boundary that return again to the boundary should be allowed. Therefore, due to the identification of points on the boundary, these orbit groups need to have a degenerated structure where the source and sink flows are crushed to one point at infinity. In general, the structure where such a source / sink pair collapses to one point is not unique, but it is defined below based on the structure of the non-degenerate singular point inside Ω and the transversal conditions at the boundary of the orbit group obtained from the intracardiac blood flow streamline visualization image data.

(Definition 3) n-bundled ss-saddle:

It is assumed that n ($\geqq$ 1) saddles are placed in the open neighborhood U of the point x $\in$ S on the sphere, and these 4n separatrices intersect the boundary of U in a transversal manner. In this situation, U is divided into 3n + 1 disjoint subdomains. Of these divided subdomains, those whose boundaries include only one saddle point have one center inside. The degenerate singular point formed by degenerating the centers and saddles to one point x is called "n-bundled source-sink-saddle" (or n-bundled ss-saddle).

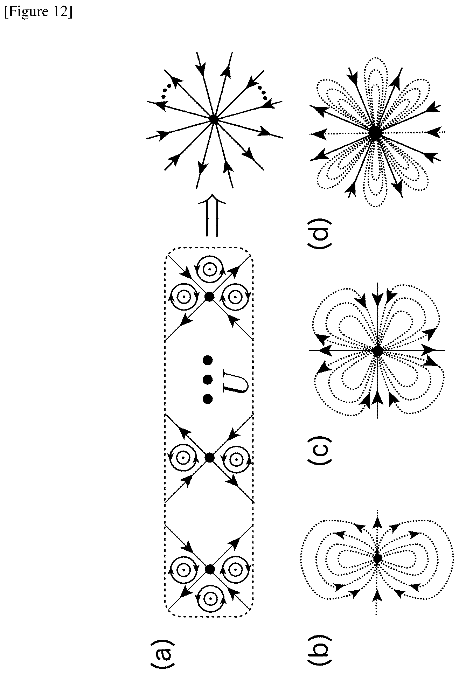

FIG. 12 illustrates the method of n-bundled ss-saddle creation. When there are n saddles satisfying the condition in the neighborhood of U near the point x, there are 2n + 2 subdomains with a center. Then, by having all these saddles and centers bundled into one point x, this degenerate singular point is constructed. From this method, the index of this degenerate singular point is 2n + 2 + (-n) = n + 2. Further, there are 4n separatrices in n-bundled ss-saddle x. Now, suppose that there are n non-degenerative saddles in the region of interest $\Omega$, and that all 4n separatrices crossing $\partial\Omega$ in a transversal manner. In this situation, if this n-bundled ss-saddle is set as a degenerate singular point at infinity on the spherical surface S obtained when $\partial\Omega$ is degenerated to one point by the equivalence relation, n + 2 + (-n) = 2 in the entire sphere without boundaries, which satisfies the Poincare-Hopf theorem, and it can be uniquely connect to the saddles and 4n separatrices from the n-bundled ss-saddle in the whole domain. When n = 1, the structure is illustrated in FIG **12**(c). When n = 2, the structure is illustrated in FIG. **12**(d), and locally, the orbit group of the source-sink pair is found in 6 of the 8 divided domains, and the other two subdomain includes source and sink orbit group.

On the other hand, if there is no saddle point inside $\Omega$ whose separatrices do not cross the boundary in a transversal manner, the flow inside $\Omega$ must be either a flow that has the source / sink structure, or a flow that all the orbit groups uniformly across the $\Omega$, in order for the orbit group to cross the boundary in a transverse manner. In this situation, the former flow becomes non-degenerate in the entire spherical surface S, if a non-degenerative source / sink point is located at infinity. On the other hand, the latter, a degenerate singular point (1-source-sink point) in which source and sink are collapsed into one at infinity as shown in FIG. **12**(b). Since this degenerate singular point corresponds to the singular point configured when n = 0 in the definition 3, it can be regarded as 0-bundled ss-saddle. Therefore, here, n-bundled ss-saddle at infinity may be considered with n $\geqq$ 0.

Finally, in order to define n-bundled ss-saddle as a singular point on an orbit, the orbits that crosses the boundaries must be "extended" to reach infinity on the plane at t $\to \pm \infty$ by identifying it with the boundary. In the actual intraventricular blood flow image data, the orbit reaches its boundary in a finite time, but collapsing the boundary into one point and placing the singular point at the north pole of the sphere corresponds to the extension of the orbit to the infinity when we consider the entire plane. With this method, the n-bundled ss-saddle can be regarded as a singular point, and the infinite number of orbits connected to the singular point can be reached in infinite time. As a result, the orbit that enters this singular point does not exit from here again, so it should be noted that the singular point is a degenerate singular point of the source / sink structure.

[New one-dimensional structure]

(Definition 4) For a proper orbit passing through x $\in$ S defined by the flow v on a surface S, the $\omega$ limit set ($\omega$-limit set) $\omega$ (x) and the $\alpha$-limit set $\alpha$ (x) are defined below.

[Equation 3]
$$\omega(x) := \cap_{n \in \mathbb{R}} \overline{\{v_t | t > n\}} \quad \text{(Limit set of } O(x), \text{ when t} \to \infty)$$

[Equation 4]
$$\alpha(x) := \cap_{n \in \mathbb{R}} \overline{\{v_t | t < n\}} \quad \text{(Limit set of } O(x), \text{ when t} \to -\infty)$$

The saddle separatrix (saddle separatrix structure) refers to an orbit in which the $\alpha$ limit set or the $\omega$ limit set is a saddle (saddle). When a saddle separatrix connects to the same saddle, this is called a self-connected saddle separatrix.

A saddle separatrix connecting different non-degenerate saddles is referred to as a heteroclinic saddle separatrix. These saddle separatrices are the elements of P (v). It has been proven that only a self-connected saddle separatrix appears when structural stability is assumed for the Hamiltonian vector field. A saddle connection diagram is a whole set of these saddles and saddle separatrices. Note that Patent Document 1 and Patent Document 2 define the same concept to include a boundary saddle ($\partial$-saddle) because there is a boundary exists in the flow domain, but it should be noted that the present description does not set an internal boundary, and therefore there is no need to think about such separatrix connecting to a boundary saddle.

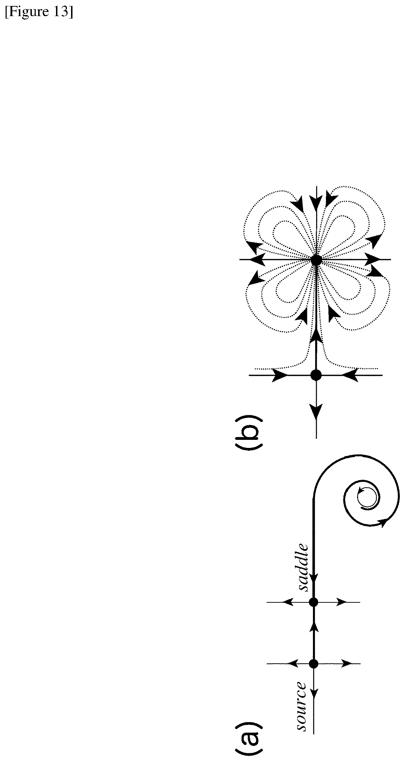

Next, a saddle separatrix associated with a compressible flow structure is defined. The ss-component refers to any one of (1) source, (2) sink, (3) non-trivial limit cycle and (4) n-bundled ss-saddle. The separatrix connecting to a saddle and to a ss-component is referred to as an ss-separatrix. FIG. **13** illustrates an example of ss-components and an ss-separatrix connecting to them. FIG. **13**(a) illustrates an example of ss-components and an ss-separatrix in $\Omega$. The separatrix connecting to the degenerate singular point n-bundled ss-saddle from the non-degenerate saddle in $\Omega$ as illustrated in FIG. **13**(b) is also an ss-separatrix. The set of saddle, saddle separatrix, ss-component, and ss-separatrix is referred to as an ss-saddle connection diagram. Hereinafter, the ss-saddle connection diagram generated by the flow v on a surface S is referred to as $D_{ss}$ (V). Here, the ss-saddle connection diagram is the one in which the boundary saddle point is removed from the one in the previous research, and the n-bundled ss-saddle is newly added.

The subject of the present description is the classification of the orbit group of the flow v on an unbounded spherical surface S with an n-bundled ss-saddle at the North Pole (infinity) of the spherical surface. The phase classification theory for characterization and tree representation of orbit groups on a spherical surface S that does not have a degenerate point at infinity (with a boundary) has been given in previous research, but here the theory is expanded to the flows below.

(Definition 5)

When the flow v on a spherical surface S without boundary satisfies the following four conditions, it is defined as a flow of finite type with an n-bundled ss-saddle.

(1) All orbits generated by the flow v are proper.
(2) All singular orbits are non-degenerate except the n-bundled ss-saddle.
(3) The number of limit cycles is finite.
(4) All saddle separatrices are either self-connected or connected to n-bundled ss-saddle.

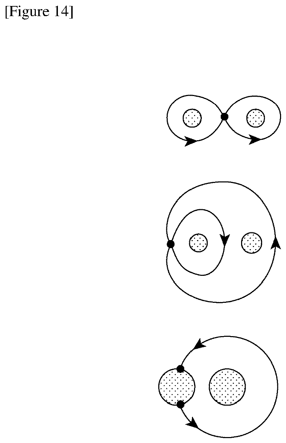

The condition of (1) has already been assumed. With the condition (2), the number of singular orbits is found to be finite and isolated. The condition (3) is an assumption that structures such as limit cycles do not accumulate infinitely. With the condition (4) and the fact that there is no boundary in the flow domain, the non-trivial limit cycle composed of the separatrices connecting the saddles has been concluded to have only three patterns illustrated in FIG. **14**. In the orbit group from intraventricular blood flow image data, there is no degenerate singular point inside the region of interest $\Omega$, and structures with infinite limit cycles or heteroclinic orbits are not observed generically; thus, application of the classification theory and intraventricular orbit group data based on this theory should not be restricted.

When the generalized Poincare-Bendixon theorem is expanded to a flow on S without boundaries with n-bundled ss-saddle as a classification theory of flow of finite type on a sphere without degenerate singular points, the following results for the limit set are obtained.

(Lemma) Let v be a flow of finite type with an n-bundled ss-saddle on a spherical surface S without boundaries. Here, the co-limit set (a-limit set) of proper non-closed orbits in v is composed of the following:

(1) saddle;
(2) sink;
(3) source;
(4) tracing (repelling) limit cycle;
(5) tracing (repelling) non-trivial limit cycle;
(6) center;
(7) n-bundled ss-saddle.

According to this lemma, the orbit group of the flow of finite type with a n-bundled ss-saddle can be classified into the following three categories:

(i) limit sets and the non-closed group orbits connecting them;
(ii) center or circuit and the periodic orbits around them;
(iii) non-closed orbits contained in intP (v)

Next, based on this, classification of the orbit groups is performed. The set of one- or smaller dimensional structures constituting the ss-saddle connection diagram $D_{ss}$ (v) of the flow of finite type v with n-bundled ss-saddle are characterized below.

(Definition 6) The set of border orbit $Bd^h(v)$ of the flow of finite: v on a surface S with n-bundled ss-saddle is given by the following.

$$\mathrm{Bd}^{h}(v) := \mathrm{Sing}(v) \cup \partial \mathrm{Per}(v) \cup \partial P(v) \cup P^{h}{}_{sep}(v)$$

Here, each set is an orbit set given below.

- $P^{h}{}_{sep}(v)$: A set of orbit set consisting of saddle separatrix and ss-separatrix in the interior point set of P (v).
- $\partial P(v)$: A set of orbits that is the boundary of a non-closed orbit set.
- $\partial \mathrm{Per}(v)$: A set of orbits that is the boundary of a periodic orbit set

Among the set of $\mathrm{Bd}^h(v)$ above, $\partial P(v)$ and $\partial \mathrm{Per}(v)$, given in previous studies, refer to limit cycle and non-trivial limit circuit. On the other hand, in the border orbit: Bd(v), the set of periodic orbits $\partial \mathrm{Per}(v)$ rotating along the boundary $\partial S$ of S, given in the previous study, is excluded in this description, since it does not consider the existence of the internal boundary. In addition, $P^{h}{}_{sep}(v)$ is a set consisting of a saddle separatrix and ss-separatrix, in addition to previous studies of $P_{sep}(v)$, and note that the separatrix connected to n-bundled ss-saddle has been added. Theorem 1 below insists that orbit groups that fill the open domain divided by $\mathrm{Bd}^h(v)$ consist only of these three shown in this FIG. 3. This result is exactly the same as the result of the previous study even if the degenerated singularity, n-bundled ss-saddle is present.

(Theorem 1) For any flow of finite type v on a sphere $S \subseteq S^2$ with a n-bundled ss-saddle, orbits in the complement set of the boundary set, $(\mathrm{Bd}^h(v))^c$ is one of the following three types.

- An open box filled with non-closed orbits in P(v). The orbit space is an open interval. FIG. 3(a) Flow in the neighborhood of ss-separatrix.
- An open annulus filled with non-closed orbits in P(v). The orbit space is the circle. FIG. 3(b) flow in the neighborhood of source (sink) disc / limit annulus.
- An open annulus filled with periodic orbits in P(v). The orbit space is an open interval. FIG. **3**(c) Flow in the neighborhood of the center disc / periodic annulus.

[New root structure]

(Root structure: $S_{\phi n}$)

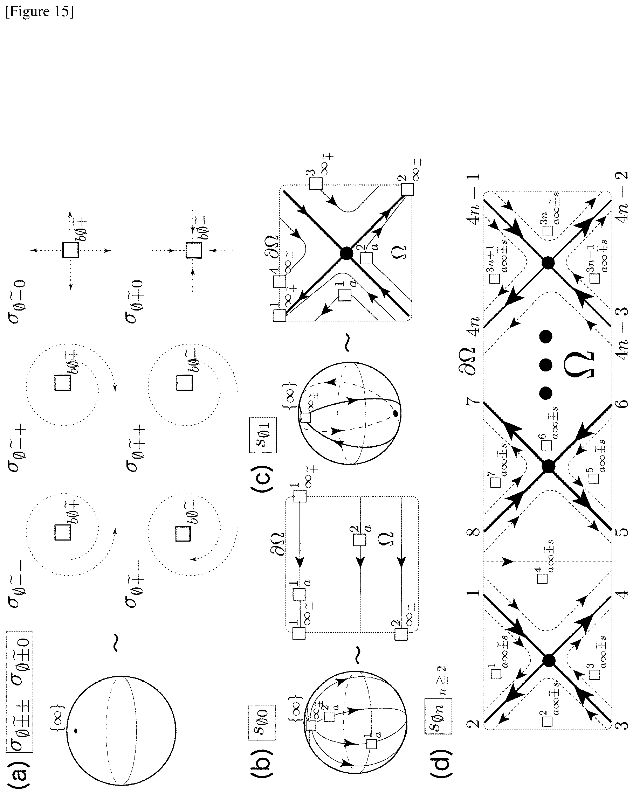

FIG. **15**(b), (c), and (d) illustrate the newly introduced root structure $S_{\phi n}$. When n-bundled ss-saddle (n $\geqq$ 0) exists at infinity $\{\infty\}$, there are n saddles in the sphere except in the neighborhood of the point at infinity. This root flow structure includes 4n separatrices connected to these saddles and n-bundled ss-saddle separatrices. In this situation, note that each separatrix is ss-saddle separatrix. Note that FIG. **15**(a) is a reprint of the root structure illustrated in FIG. **5**.

When n = 0, ss-saddle separatrix does not exist, so saddle does not need to exist in S \ {oo}. In this situation, the flow generated by the 0-bundled ss-saddle at infinity illustrated in FIG. **12**(b) has a flow structure in S \ {∞} and in the ROI of the domain $\Omega$ as illustrated in FIG. **15**(b). Each orbit can contain a class-a orbit structure connecting to the point at infinity and a class-$\infty_{\sim\pm}$ orbit structure that degenerates to the point at infinity {oo}, as is discussed later. In order to explain this situation in detail, let us consider expanding the flow on $\Omega$ as illustrated in FIG. **15**(b). There is a uniform flow from one side boundary $\partial\Omega$ of the region of interest $\Omega$ to the other side boundary. There can be degenerate structures of class-$\infty_{\sim\pm}$ at the starting point and the ending point, but any s (s $\geqq$ 0) structures of class-a can be attached between them. Therefore, in order to express these structures, it is necessary to collectively express the source / sink structure of the start point and the end point at $\partial\Omega$ and the orbit of class-a connecting between them. For example, the representation $[\square^1_{\infty\sim+}, \square^1_a, \square^1_{\infty\sim-}]$ for the top of the orbit structure shown in the figure, and represent one degenerated structure as one "set". In general, with respect to one orbit structure, triplet

$$\square_{a\infty\sim\pm} := [\square_{\infty\sim+}, \square_{as}, \square_{\infty\sim-}]$$

is used for the expression.

$\square_{as}$ represents the orbit structure $\square_a$ of s (s $\geqq$ 0) class-a connecting between $\square_{\sim+}$ and $\square_{\sim-}$, and are ordered as follows.

$$\square_{as} := \square^1_a \ldots \ldots \cdot \square^s_a \ (s > 0), \ \square_{as} = \lambda_{\sim} \ (s = 0)$$

Here, this triplet does not have a structure that fills within $\square_{\infty\sim\pm}$, that is, if there is no structure that degenerates at infinity, the symbols $\infty_{\sim\pm}$ are used, double-sign in the same order, and if there is no class-a, the symbol $\lambda_{\sim}$ is used.

It is expressed as follows that this triplet structure is further arranged in s pieces.

$$\Box_{a\infty \sim \pm s} := \Box^1{}_{a\infty \sim \pm} \cdot \ldots \ldots \cdot \Box^s{}_{a\infty \sim \pm} \ (s > 0), \ \Box_{a\infty \sim \pm s} = \lambda_\sim \ (s = 0)$$

By reading and arranging the orbit structure inside $\Omega$ created in this way in an order from 0-bundled ss-saddle (from top to bottom in the figure), the COT representation of this root structure is $s_{\phi 0}$ ($\Box_{a\infty \sim \pm s}$) can be given.

When n = 1, the 1-bundled ss-saddle is connected to the separatrix of a saddle in S\{oo}, which is the separatrix from infinity {$\infty$}, as illustrated in FIG. 15(c). In the region of interest, four ss-saddle separatrices extending from the saddle inside the $\Omega$ intersect $\partial\Omega$ across transversely. Note that for each separatrix, the orbit structure of class-$\infty_{\sim\pm}$ can be degenerated to {$\infty$} on a sphere or to $\partial\Omega$ in the region of interest. Further, the spherical surface S (or $\Omega$) is divided into 3n + 1 = 4 regions by this separatrix, and in each divided region, there are such orbit structures corresponding to the source sink pair that starts from the 1-bundled ss-saddle and returned to it. For each orbit in this divided domain, the orbit structure $\Box_{a\infty\sim\pm}$ can be considered.

Unlike the case of n = 0, this orbit has a special topological orbit called ss-saddle separatrix that connects to the saddle, so the order using these orbit structures in the COT representation be decided as follows. Now, arbitrarily select one ss-saddle separatrix that is connected to the saddle. Then, consider the structure $\Box_{\infty\sim\pm}$ in which this separatrix has degenerated to the crossing point on $\partial\Omega$, and the order of the orbit structure $\Box_{a\infty\sim\pm}$ in the divided domain in the counterclockwise direction. Here, for an explanation, consider the case when the selected separate structure is a source, that is, the case with a structure degenerated to $\Box_{\infty\sim+}$ at the crossing point on boundary $\partial\Omega$ with this separatrix. In this case, there is another separatrix (another one on the counterclockwise side of the selected separatrix) that is the other boundary of this divided domain, and note that it is a degenerate to the structure $\Box_{\infty}$at the point where it crosses the boundary $\partial\Omega$. The orbit group with a topological structure in this divided domain is written as $\Box^1{}_{a\infty\sim\pm s}$, and the orbit groups belonging to the orbit group are ordered based on the distance from the orbit of separatrix connecting two saddles and the infinity. That is, they are ordered from a point close to the saddle toward the boundary $\partial\Omega$ of the region of interest. Performed this operation successively to the remaining separatrix and the orbit group with a topological structure in the divided domains (Write them $\Box^k{}_{a\infty\sim\pm s}$, and k = 2, 3, 4), and ordered them in counterclockwise; then, the following COT representation can be obtained.

$$S_{\phi 1} \left\{ \Box^1{}_{a\infty \text{ to} \pm s}, \ \Box^2{}_{a\infty\sim\pm s}, \ \Box^3{}_{a\infty\sim\pm s}, \ \Box^4{}_{a\infty\sim\pm s} \right\}$$

Note that only this root structure $s_{\phi 1}$ has a cyclic degree of freedom in a method of the selection of the first separatrix, so it is enclosed in { } in COT representation.

When n $\geqq$ 2, the spherical surface S is divided into 3n + 1 by the separatrix connecting the n-bundled ss-saddle and the saddle, and the two-dimensional domain is the same as in FIG. 15(a) in each domain. The orbit structure represented by $\Box_{a\infty\sim\pm s}$ is still included, but in the 2n + 2 domain, the orbit structure is internally connected to the source and sink pairs, while in the remaining n-1 domain, the orbit has a structure starting from the source, passing around the spherical surface, and ending into the sink on the opposite side. In addition, the separatrix that connects 4n n-bundled ss-saddles and n saddles can embed class-$\infty_{\sim\pm}$ orbit that degenerates to the infinity.

A method of giving a COT representation (FIG. 15(d)) that expresses the positional relationship of these orbit structures without ambiguity will be described using the region of interest $\Omega$. First, consider the separatrix attached to each saddle and the domains divided by the separatrix, and the structures from the one at the end (left end in the figure) of the saddles are numbered. That is, the leftmost separatrix is numbered 1 to 4 counterclockwise as shown in FIG. 15(d), and the three domains surrounded by them are also numbered 1 to 4 counterclockwise in order. Next, the four separatrices from the right side saddle are numbered 5 to 8 counterclockwise in order from the lower left one, and the three domains surrounded by them are numbered 5 to 7 in order. Inductively, for k $\geqq$ 2, the saddle separatrices on the right side are numbered 4k + 1 to 4k + 4 counterclockwise from the lower left, and three domains surrounded by them are numbered 3k - 1 to 3k + 1. This operation of numbering the separatrices and its dividing domains, is repeated up to the nth saddle, and the orbit structures are expressed in the order of the numbers. In the divided 3n + 1 domains, uniform flow orbit group with any s $\geqq$ 0 or orbit group ($\Box^k{}_{a\infty\sim\pm s}$, k= 1, ..., 3n + 1) can exist. Inside the 2n + 2 domains that have orbit structures with starting from and an ending at the connected component of the boundary $\partial\Omega$, a triplet structure is located from the structure close to the saddle toward the boundary as in $s_{\phi 1}$, and inside the n-1 domains that have orbit structures with starting from and ending at non-connected component of the boundary $\partial\Omega$, a triplet structure is sequentially ordered from the saddle on the right side to the saddle on the left side; then, the COT representation with this root structure can be given below.

$$S_{\varphi n} \left( \Box^1{}_{a\infty\sim\pm s}, \ \Box^2{}_{a\infty\sim\pm s}, \ \Box^3{}_{a\infty\sim\pm s}, \ \Box^4{}_{a\infty\sim\pm s}, \ \Box^5{}_{a\infty\sim\pm s}, \ \Box^6{}_{a\infty\sim\pm s}, \ \Box^7{}_{a\infty\sim\pm s}, \ ..., \ \Box^{3n-1}{}_{a\infty\sim\pm s}, \ \Box^{3n}{}_{a\infty\sim\pm s}, \right.$$
$$\left. \Box^{3n+1}{}_{a\infty\sim\pm s} \right)$$

Then, classification of one-dimensional structure of Bd$^h$(v) constructing the ss-saddle connection diagram $D_{ss}$(v) in a flow of finite type: v with n-bundled ss-saddle on a surface S, and its corresponding COT representation are given. As described above, Bd$^h$(v): = Sing (v) $\cup\partial$Per (v) $\cup\partial$P (v) UP$^h_{sep}$(v), 0-dimensional / 1-dimensional structures that realizes Bd$^h$(v) corresponding to each set are introduced. However, it should be noted that to which set each one-dimensional structure belongs depends on the orbit group around it.

(0-dimensional structure: $\infty_{\sim\pm}$ )

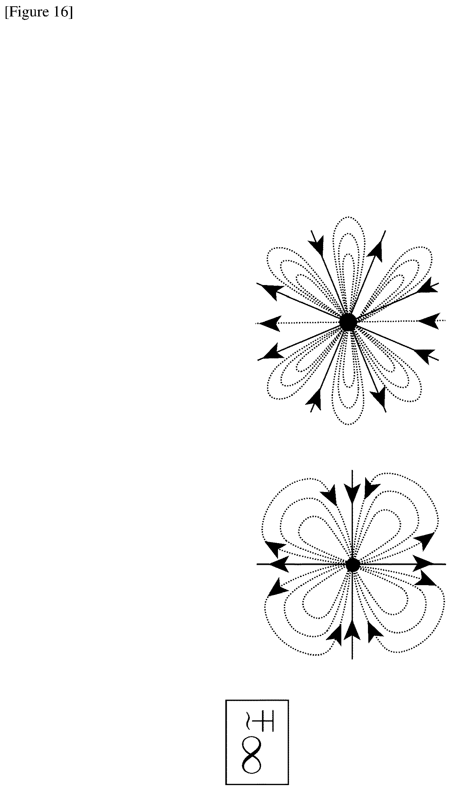

This is a degenerated singular point corresponding to the n-bundled ss-saddle at infinity $\{\infty\}$ on the sphere. FIG. 16 illustrates 1-bundled ss-saddle and 2-bundled ss-saddle, as examples, but in general, it can be applied to n-bundled ss-saddle representation. Since the information on which n actually corresponds is given in the COT representation $s_{\phi n}$ of the root structure and since only one degenerated singular point exists in the structure, it is not necessary to be identified as a symbol. This structure is in class-$\infty_{\sim\pm}$ .

(One-dimensional structure: $a_{\sim\pm}$ , $q_{\sim\pm}$ )

$a_{\sim\pm}$ and $q_{\sim\pm}$ are structures corresponding to the above-mentioned (S3). In this situation, since the saddle has the same source / sink structure, it becomes slidable saddle. The structure is classified according to the positional relationship of the ss-component connected to this slide saddle. In this situation, since all neighborhood orbits are the two-dimensional domain (open box) filled with non-closed orbits, these one-dimensional structures always be an element of P$^h_{sep}$(v).
First, the structures corresponding to the slidable saddle in FIG. 11(a) outside the ss-component to which the slidable saddle is connected, is $a_{\sim\pm}$. This structure has two source / sink structures that connect to the saddle. If both of them are connected to n-bundled ss-saddle $\{\infty\}$, they are the saddles that form the root structure, so they cross the boundary $\partial\Omega$ transversely of the region of interest $\Omega$ and a COT representation to it need not be assigned. When only one side is connected to n-bundled ss-saddle, this is a structure that degenerates to infinity (or $\partial\Omega$) when the representation $a_{\sim\pm}\{\Box_{\sim\pm}$ , $\infty_{\sim\pm}$ } is assigned. Therefore, the structures $a_{\sim\pm}$ belong to both class-$\sim\pm$ and class-$\infty_{\sim\pm}$. Since the order of these source / sink structures can be freely selected, the COT representation is $a_{\sim\pm}$ {$\Box_{\sim\pm}$ , $\Box_{\sim\pm}$ }, with double-sign in the same order.
Next, the structure corresponding to the slidable saddle in FIG. 11(b) inside the ss-component to which the slidable saddle is connected, is $q_{\sim\pm}$. This COT representation is $q_{\sim\pm}$ ($\Box_{\sim\pm}$ ) with double-sign in the same order. Since this structure is contained only in $b_{\sim\pm}$ , it is not connected to the n-bundled ss-saddle. Therefore, it belongs to class-$a_{\sim\pm}$.
Table 2 summarizes the internal structural sets used in the COT representation of flow of finite type: v on a sphere S with n-bundled ss-saddle (no boundaries). Table 3 summarizes the structures constituting the flow of finite type: v on the spherical surface S (without physical boundaries) with n-bundled ss-saddle and their COT representation.

[Table 2]

| Structure set | Structure group symbols | Structure orbit group | Noted |
|---|---|---|---|
| class-$b_+$ | $\Box_{b+}$ | $\{b_{\mp}, b_+\}$ | Two-dimensional structure |
| class-$b_-$ | $\Box_{b-}$ | $\{b_{\mp}, b_-\}$ | Two-dimensional structure |
| class-$b_{\tilde{\pm}}$ | $\Box b_{\tilde{\pm}}$ | $\{b_{\mp}\}$ | Two-dimensional structure |
| class-$\tilde{+}$ | $\Box_{\tilde{+}}$ | $\{p_\pm, b_{\pm\pm}, b_{\pm\mp}, q_\pm, a_{\tilde{+}}, \sigma_{\tilde{+}\pm}\sigma_{\tilde{+}0}\}$ | Source structure |
| class-- | $\Box_{\tilde{-}}$ | $\{p_\pm, b_{\pm\pm}, b_{\pm\mp}, q_\pm, a_{\tilde{-}}, \sigma_{\tilde{-}\pm}, \sigma_{\tilde{-}0}\}$ | Sink structure |
| class-$\alpha_+$ | $\Box_{\alpha+}$ | $\{p_{\tilde{+}}, b_{++}, b_{+-}, q_+, \sigma_+\}$ | Exists only in two-dimensional structure $b_+$ |
| class-$\alpha_-$ | $\Box_{\alpha-}$ | $\{p_{\tilde{-}}, b_{-+}, b_{--}, q_-, \sigma_-\}$ | Exists only in two-dimensional structure $b_-$ |
| class-$a$ | $\Box_a$ | $\{a_\pm\}$ | |

(continued)

| Structure set | Structure group symbols | Structure orbit group | Noted |
|---|---|---|---|
| class-$a_-^{\sim}$ | $\square_{a\text{-}}^{\sim}$ | $\{q_{\simeq}, a_{\pm}\}$ | |
| class-$a_+^{\sim}$ | $\square_{a+}^{\sim}$ | $\{q_{\widetilde{\mp}}, a_{\pm}\}$ | |
| class-$\infty_+^{\sim}$ | $\square_{\infty+}^{\sim}$ | $\{a_{\widetilde{\mp}}\}$ | Structure degenerated to $\partial\Omega$ |
| class-$\infty_-^{\sim}$ | $\square_{\infty\text{-}}^{\sim}$ | $\{a_{\simeq}\}$ | Structure degenerated to $\partial\Omega$ |

[Table 3]

| Root structure | COT representation |
|---|---|
| $\sigma_{\emptyset\widetilde{\pm}\pm}, \sigma_{\emptyset\widetilde{\pm}0}$ | $\sigma_{\emptyset\widetilde{\mp}\pm}(\square_{b\emptyset\widetilde{\pm}}), \sigma_{\emptyset\widetilde{\mp}0}(\square_{b\emptyset\widetilde{\pm}})$ |
| $s_{\emptyset0}$ | $s_{\emptyset0}(\square_{a\infty\widetilde{\pm}s})$ |
| $s_{\emptyset1}$ | $s_{\emptyset1}\{\square^1_{a\infty\widetilde{\pm}s}, \square^2_{a\infty\widetilde{\pm}s}, \square^3_{a\infty\widetilde{\pm}s}, \square^4_{a\infty\widetilde{\pm}s}\}$ |
| $s_{\emptyset n}$ $(n \geqq 2)$ | $s_{\emptyset n}\left(\square^1_{a\infty\widetilde{\pm}s}, \ldots, \square^{3n+1}_{a\infty\widetilde{\pm}s}\right)$ |
| Two-dimensional structure | COT representation |
| $b_{\widetilde{\pm}}$ | $b_{\widetilde{\pm}}(\square_{\widetilde{\mp}}, \{\square_{a\widetilde{\pm}s}\})$ |
| $b_{\pm}$ | $b_{\pm}(\square_{\alpha\pm})$ |
| Singular structure | COT representation |
| $\sigma_{\pm}$ | $\sigma_{\pm}$ |
| $\sigma_{\widetilde{\pm}\pm}, \underline{\sigma_{\widetilde{\pm}0}}$ | $\sigma_{\widetilde{\mp}\pm}, \sigma_{\widetilde{\mp}0}$ |
| Cycles | COT representation |
| $p_{\widetilde{\pm}}$ | $p_{\widetilde{\mp}}(\square_{b\widetilde{\pm}})$ |
| $p_{\pm}$ | $p_{\pm}(\square_{b\pm})$ |
| Circuits | COT representation |
| $a_{\pm}$ | $a_{\pm}(\square_{b\pm})$ |
| $q_{\pm}$ | $q_{\pm}(\square_{\widetilde{\mp}}, \square_{\simeq}, \square_{as})$ |
| $b_{\pm\pm}$ | $b_{\pm\pm}\{\square_{b\pm}, \square_{b\pm}\}$ |
| $b_{\pm\mp}$ | $b_{\pm\mp}(\square_{b\pm}, \square_{b\mp})$ |

(continued)

| Slidable saddle | COT representation |
|---|---|
| $a_{\overline{\pm}}$ | $a_{\overline{\pm}}\{\square_{\overline{\pm}}, \square_{\overline{\pm}}\}$ |
| $q_{\overline{\pm}}$ | $q_{\overline{\pm}}(\square_{\overline{\pm}})$ |

[Tree representation]

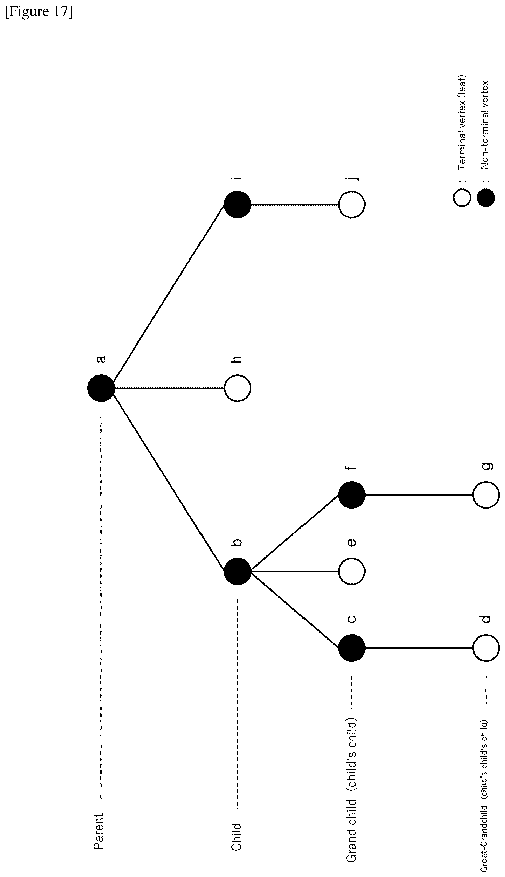

Next, with reference to FIG. 17, the basic matters regarding the "tree representation" used in the present description will be described. FIG. 17 shows an example of a general tree representation. As illustrated, the tree representation is a graph with a structure in which the vertices are connected with a line. The vertices of trees are roughly divided into two types: those located at the end of the tree (∘) and those not located at the end (●). The former (d, e, g, h, j) is called a terminal vertex ("leaf" or "leaf"), and the latter (a, b, c, f, i) is called a non-terminal vertex. The non-terminal vertex (a) at the top is called the "root". Of the two vertices directly connected by a line, the one closer to the root (upper in the figure) is called the "parent" and the one closer to the leaf is called the "child". The root is the only parentless vertex in the tree representation. Vertices other than the root always have only one parent. For example, in FIG. 17, b is a child of a and a parent of c, d is a child of c, and so on.

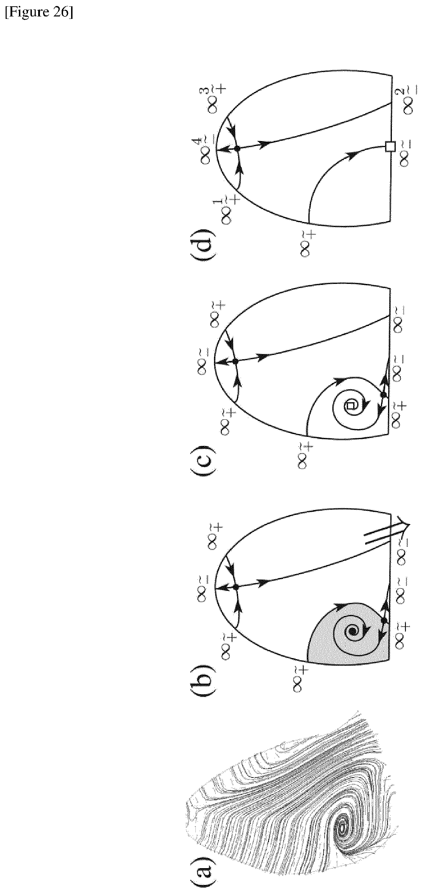

In the word representation described herein, the root is the outermost structure in the flow pattern of interest, and specifically includes any of a vortex center, a sink, and a source. The leaves are the structures inside the root. As will be described later, in the present technology, when a target flow pattern is given, first, the outermost structure corresponding to the root is determined. Next, the innermost structure of the internal structures corresponding to the leaves is determined, letters are given, and this structure is extracted. Next, the steps of determining the innermost structure among the remaining structures, giving characters, and pulling out this structure are repeated until the root of the outermost structure is reached. For example, when the flow pattern shown in FIG. **26**(a) as described below is given, COT representation,

$$s_{\varphi 1}\{[\infty_{\sim +}, \lambda_{\sim}, a_{\sim}\{\sigma_{\sim\_\_}, \infty_{\sim\_}\}], \lambda_{\sim}, \lambda_{\sim}, \lambda_{\sim}\}$$

is obtained. In this case, the root is $s_{\varphi 1}$, leaves are $\sigma_{\sim\_}, \infty_{\sim}, a_{\sim}, \infty_{\sim +}$ in an order from the inner side.

[First Embodiment]

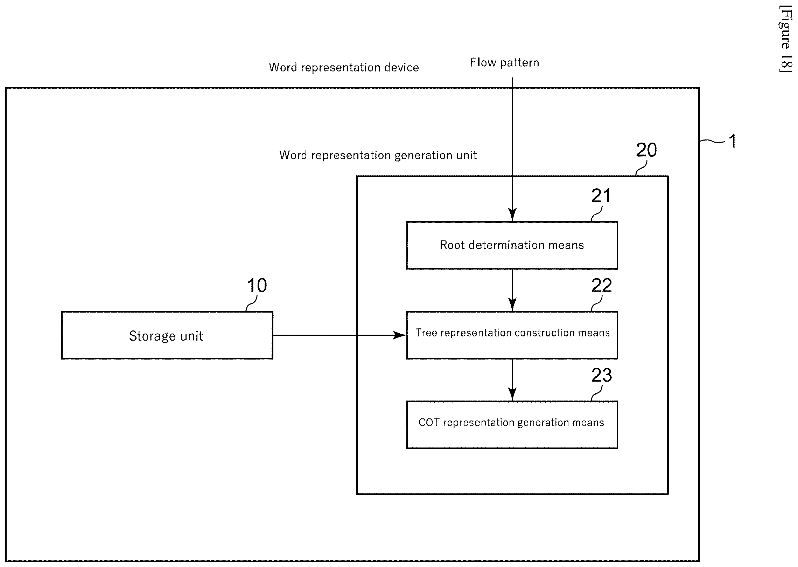

The first embodiment of the present invention is a word representation device that expresses the streamline structure of a flow pattern in a two-dimensional domain. FIG. **18** shows a functional block diagram of the word representation device 1 according to the first embodiment. The word representation device 1 includes a storage unit 10 and a word representation generation unit 20. The word representation generation unit 20 includes a root determination means 21, a tree representation construction means 22, and a COT representation generation means 23.

The storage unit 10 stores the correspondence between each streamline structure and its character (COT representation) with respect to the plurality of streamline structures constituting the flow pattern. This correspondence is summarized in, for example, Tables 1 and 3. That is, Table 1 shows the relationship between the streamline structure given in the previous study and the COT representation, and Table 3 shows the relationship between the streamline structure on a sphere with n-bundled ss-saddle given in the present description and the COT representation.

The root determining means 21 determines a root for expressing a given flow pattern as a tree. Specifically, it is determined which of the outermost structures shown in Tables 1 and 3 corresponds to the flow pattern. In this situation, the direction of rotation of the flow is the direction of rotation when the singular point or boundary as the root is viewed as the center. The "internal structure" of a structure is the connected component of the complement that does not contain a root. Further, the "innermost structure" refers to a structure having no internal structure or a structure having no internal structure other than a flow box (rectangle consisting of orbits in the shape of an open section).

The streamline structure constituting the flow pattern includes a root structure on a surface with an n-bundled ss-saddle degenerated singular point. Typically, such a flow pattern is a pattern of blood flow in the ventricles.

The tree representation generating means 22 extracts the streamline structure of the given flow pattern, assigns characters to the extracted streamline structure based on the correspondence relationship stored in the storage unit 10, and obtains the extracted streamline structure. A tree representation of a given flow pattern is constructed by repeating the process of deleting from the innermost part of the flow pattern until the root is reached. The construction of the tree representation is carried out based on the following principles.

1. From the given flow pattern, streamline structures are extracted one by one in order from the innermost part.
2. When extracting a streamline structure, a character (COT representation) corresponding to the streamline structure is added as the apex of the tree. Then, the streamline structure is deleted. Hereinafter, the process of "extracting the streamline structure from the flow pattern, adding characters to the streamline structure, and deleting the streamline structure" is collectively referred to as "extracting the structure".
3. When extracting the structure, the process starts by extracting the innermost structure and then pulling out in sequence until all the structures are gone.

3.1. The innermost structure corresponds to the leaf.
3.2. The structure to be pulled out last corresponds to the root. That is, the process of pulling out the structure is repeated until the root of the given flow pattern is reached.
3.3. When pulling out a structure, it is replaced with □ and link □ with that structure to correspond (so that when pulling out the upper structure containing this □, this structure can be the "child" of the upper structure).

By executing the above processing, the tree representation construction means 22 outputs the characters corresponding to all the streamline structures included in the given flow pattern as a tree representation.

The COT representation generation means 23 converts the tree representation configured by the tree representation construction means 22 into a COT representation. Specifically, the COT representation is constructed by converting the tree representation into a representation using parentheses. However, the conversion is realized by using curly braces {} when the elements are placed in a circular order, and by using parentheses () when the elements are placed in a total order. Later, the generation of the COT representation will be described with reference to an example. For example, the topological data structure shown in FIG. **26**(b) is extracted from the flow pattern shown in FIG. **26**(a). The topological data structure of FIG. **26**(b) minus the innermost streamline structure is shown in FIG. **26**(c). Hereinafter, by repeating a process of pulling out the structure according to the procedure described above, COT representation

$$s_{\varphi 1} \{ [\infty_{\sim+}, \lambda_{\sim}, a_{\sim} \{ \sigma_{\sim}, \infty_{\sim} \} ], \lambda_{\sim}, \lambda_{\sim}, \lambda_{\sim} \}$$

is obtained.

As described above, one of the examples of the word representation device according to the present embodiment represents the streamline structure of the flow pattern including the root structure on a surface with an n-bundled ss-saddle degenerated singular point in words. Therefore, the word representation device according to the present embodiment represents the streamline structure of the flow pattern including the flow generated by the moving physical boundary in words.

According to the present embodiment, when a flow pattern in a two-dimensional domain is given, a COT representation of the flow pattern can be obtained, that is, the flow pattern can be converted into characters.

In the embodiment described above, the characters corresponding to the streamline structure are alphabets and Greek letters with subscripts added. However, the character corresponding to the streamline structure is not limited to this, and can be any kind of character, and can be a one like pictogram (the same applies hereinafter).

[Second Embodiment]

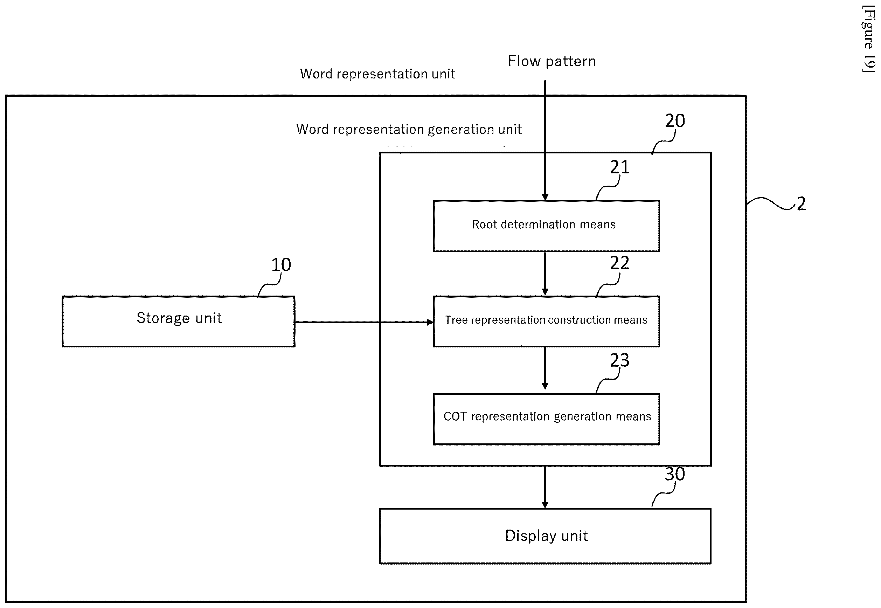

FIG. **19** illustrates a functional block diagram of the word representation device 2 according to the second embodiment. The word representation device 2 includes a display unit 30 after the word representation generation unit 20 in addition to the configuration of the word representation device 1 in FIG **18.** Other configurations and operations are common to the word representation device 1.

The display unit 30 displays the topological data structure extracted from the flow pattern. The display unit 30 may further display the COT representation generated by the COT representation generation means 23 in addition to the topological data structure. The display unit 30 may further fill and display the vortex flow domain corresponding to the COT representation. The display unit 30 may further illustrate the flow pattern itself in the region of interest by displaying the streamline extracted from the original image of the flow and its singular point. Further, when the flow pattern is that of intraventricular blood flow, the display unit 30 may further display an echocardiography VFM (vector flow mapping) image or a blood flow velocity vector.

According to this embodiment, the topological data structure of the flow pattern can be visualized.

[Third Embodiment]

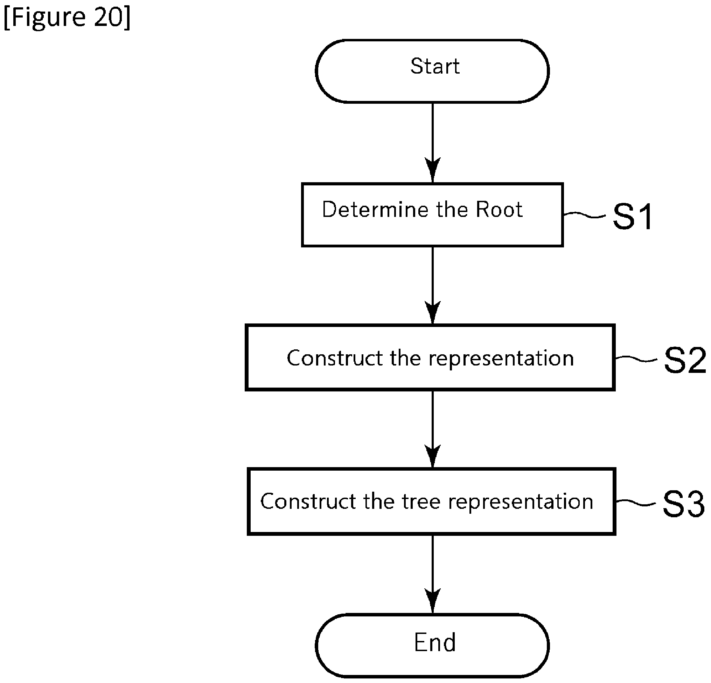

The third embodiment of the present invention is a word representation method for expressing the streamline structure of a flow pattern in a two-dimensional domain. This method is performed by a computer equipped with a storage unit and a word representation generation unit. FIG. **20** shows the flow of the word representation method according to the third embodiment. The method includes a step S1 for determining the root of a given flow pattern, a step S2 for constructing a tree representation of the flow pattern, and a step S3 for generating a COT representation of the flow pattern. The streamline structure constituting the flow pattern includes a root structure on a surface with an n-bundled ss-saddle degenerated singular point.

In step S1, the method determines the root of a given flow pattern. Since the specific processing of the root determination is the same as that described in the first embodiment, detailed description thereof will be omitted.

In step S2, the method constructs a tree representation of a given flow pattern.

In step S3, the method converts the tree representation configured in step S2 into a COT representation.

According to the present embodiment, when a flow pattern in a two-dimensional domain is given, a COT representation of the flow pattern can be obtained, that is, the flow pattern can be converted into characters.

[Fourth Embodiment]

A fourth embodiment of the present invention is a program for a process executed by a computer including a storage unit and a word representation generation unit. This program makes the computer execute the flow shown in FIG **20**. That is, this program makes the computer execute step **S**1 for determining the root of the given flow pattern, step S2 for constructing the tree representation of the flow pattern, and step S3 for generating the COT representation of the flow pattern. The streamline structure constituting the flow pattern includes a root structure on a surface with an n-bundled ss-saddle degenerated singular point.

According to the present embodiment, when a flow pattern in a two-dimensional domain is given, a program for obtaining a COT representation of the flow pattern, that is, characterizing the flow pattern can be implemented in software, so that a computer can be used. It is possible to realize highly accurate word representations.

[Example of structure extraction algorithm]

Hereinafter, the procedure for giving a word representation to the blood flow pattern will be specifically described based on an example of intraventricular blood flow. First, the root, which is the outermost structure of the flow pattern, is determined before executing the following algorithm. The extraction algorithm of the discrete combination structure such as character generation and tree structure by classifying the orbit group data of the intraventricular blood flow image is roughly divided into the following three steps ((N1) to (N3)). Through these steps, it is possible to provide a one-to-one correspondence link representation and COT representation for the topological structure of the orbit group data of the intraventricular blood flow image. These steps are executed by the word representation generation unit 20 in the word representation devices 1 and 2 of FIG.s **18** and **19.**

(N1) A step of performing topological preconditioning on the orbit group data of an intraventricular blood flow image to form an ss-saddle connection diagram.
(N2) A step of extracting a link structure from the orbit structure of the orbit group data of the intraventricular blood flow image.
(N3) A step of constructing a COT representation and an accompanying tree representation from the orbit structure of the orbit group data of the intraventricular blood flow image.

The algorithm of each process will be described below.

(N1) Extraction Algorithm for ss-saddle connection diagram.

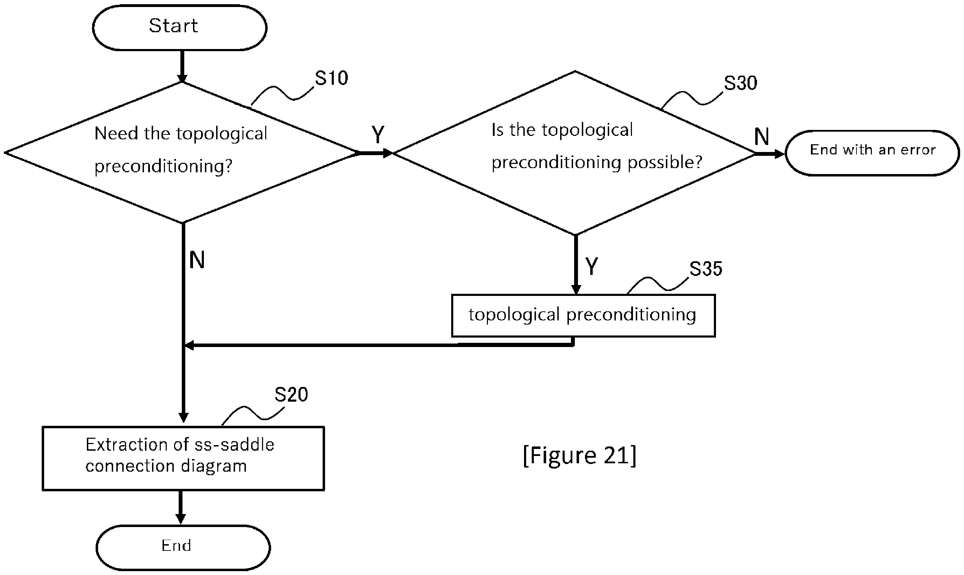

FIG. **21** is a flow chart showing the processing of step N1. A ss-saddle connection diagram is constructed for the orbit group data of the intraventricular blood flow image in the region of interest. In this situation, adding the minimum necessary number of saddles so as not to contradict the Poincare-Hopf index theorem by performing topological preconditioning can be allowed as necessary. For patterns for which topological preconditioning is not possible, subsequent analysis cannot be performed, so an error is returned. This can be achieved, for example, by the following steps (S10) to (S30).

(S10) Confirm whether topological preconditioning is required for the intraventricular blood flow image in the region of interest. If the topological preconditioning is unnecessary, the process proceeds to (S20).

If topological preconditioning is required, the process proceeds to (S30).

(S20) The ss-saddle connection diagram is extracted, and this step is normally completed.

Proceed to the link structure extraction step (N2).

(S30) If the topological preconditioning is possible on the image, the topological preconditioning (S35) is performed and the process proceeds to (S20). If topological preconditioning is not possible on the image, it ends with an error.

(N2) Link Structure Extraction Algorithm.

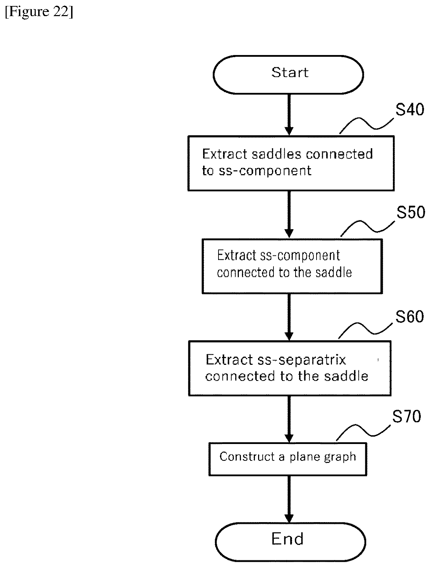

FIG. 22 is a flow chart showing the processing of step N2. This is a step of extracting as a graph structure what kind of ss-component the ss-separatrix connected to the saddle is connected to from the ss-saddle connection diagram configured by (N1). In fact, the vertices are "saddle connected to ss-component" and "ss-component connected to saddle", and the sides are ss-separatrix connected to a saddle. This graph is a planar graph in which the number of vertices and edges is finite. Therefore, this graph is extracted. For example, there are the following methods for extracting this graph.

(S40) Extraction of the saddle connected to the ss-component.

(S50) Extraction of the ss-component connected to the saddle.

(S60) Extraction of the ss-separatrix connected to the saddle.

(S70) Construction of the planar graph by aligning vertices and sides corresponding to the order in the plane from the abstract graph obtained from this information.

As shown in FIG s. **24**(a) and (b), there are cases where they have the same COT representation but different orbit topologies. In this case, if the connection relationship of separatrix is maintained as a link structure, correct restoration can be performed by defining the connection relationship with reference to the ss-saddle connected diagram when reconstructing the ss-saddle connected diagram from the COT representation.

(N3) Algorithm for generating tree representation and COT representation.

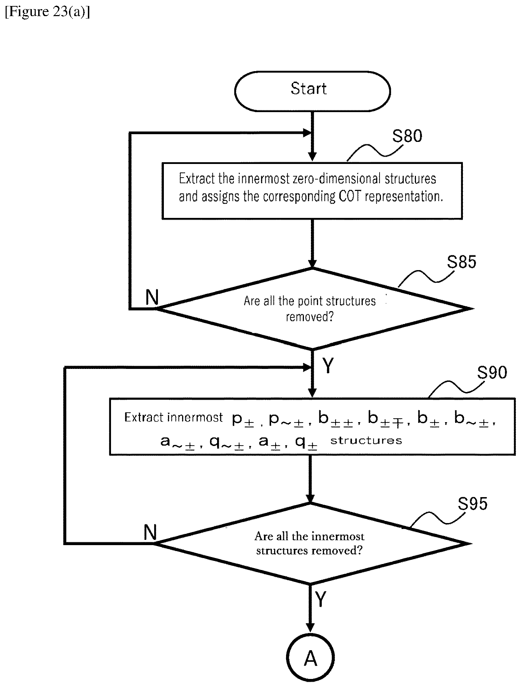

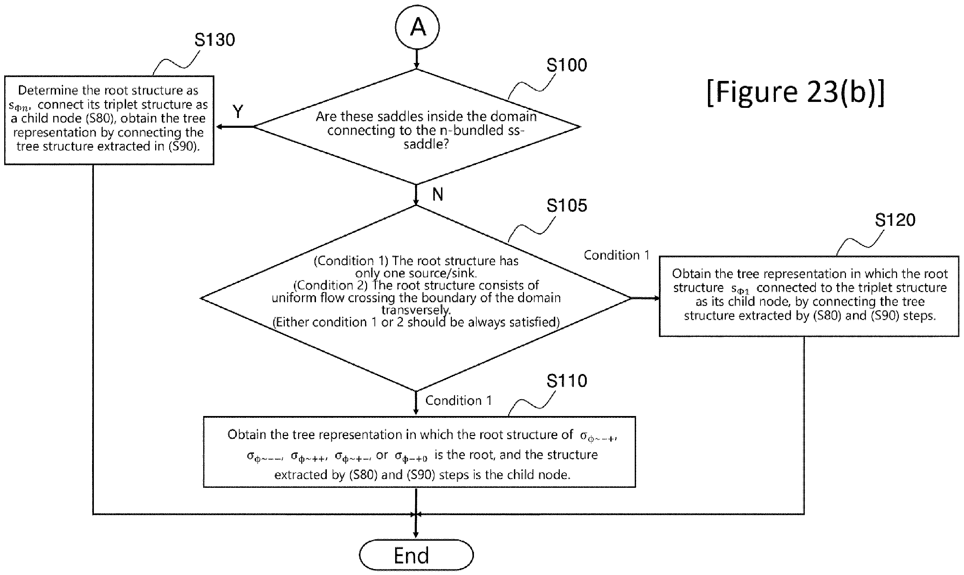

FIG. **23** is a flow chart showing the processing of step N3. (a) is the first half, and (b) is the second half. As in previous studies, the algorithm for giving the COT representation and the associated tree structure to the topological structure of the orbit group of the intraventricular blood flow image is summarized as follows.

(S80) The innermost 0-dimensional structure is extracted and the COT representation associated therewith is assigned. Point structures as COT representations (characters) are assigned, $\sigma_{\sim++}$ for a source with counterclockwise rotation; $\sigma_{\sim+-}$ for a source with clockwise rotation; $\sigma_{\sim+0}$ for source with no rotation; $\sigma_{\sim-+}$ for a sink with counterclockwise rotation; $\sigma_{\sim--}$ for a sink with clockwise rotation; $\sigma_{\sim-0}$ for a sink with no rotation; $\sigma_{+}$ for a vortex center with the periodic orbit counterclockwise; $\sigma_{+}$ for a vortex center with the clockwise rotation. After assigning the COT representation, remove the structure from the orbit structure and replace it with the label □.

(S85) Whether or not all the point structures have been deleted as a result of the processing in (S80) is determined. When all the point structures are deleted, the process proceeds to (S90). If the point structure remains, the process returns to (S80).

(S90) In the innermost domain, all the structures including $p_{\pm}$, $p_{\sim\pm}$, $b_{\pm\pm}$, $b_{\pm\mp}$, $b_{\pm}$, $b_{\sim\pm}$, $a_{\sim\pm}$, $q_{\sim\pm}$, $a_{\pm}$, $q_{\pm}$ are extracted. If these structures are found, remove them from $\Omega$ and replace them with □, which indicates that there is no structure inside. Since in the structure extracted in this situation, there is an internal orbit structure that was once removed and replaced with □, an edge is generated so that internal orbit structure is regarded as its own "child node" and internal orbit structure itself is regarded as a "parent node".

(S95) As a result of the processing in (S90), whether or not all the innermost structures have been deleted is determined. When all the innermost structures are deleted, the process proceeds to (S100). If the innermost structure remains, the process returns to (S90).

(S100) As a result of (S90), all orbit structures are removed from the inside of the $\Omega$. In this situation, if there is no saddle connected to the n-bundled ss-saddle inside the $\Omega$, and if the root structure has only one source / sink, the process proceeds to (S110), if there is only uniform flow crossing the boundary of the region of interest, the process proceeds to (S120). If there is a saddle inside the $\Omega$, the process proceeds to (S130).

(S105) Which of the following (Condition 1) and (Condition 2) is satisfied, is determined.

(Condition 1) The root structure has only one source / sink.

(Condition 2) The root structure consists of a uniform flow that crosses the boundary of the region of interest transversely.

If (Condition 1) is satisfied, then the process proceeds to (S120). If (Condition 2) is satisfied, the process proceeds to (S110). Note that either condition 1 or condition 2 is always satisfied.

(S110) The root structure is a flow on a spherical surface having no degenerated singularity. In this situation, the root structure is determined according to the $\Omega$ internal structure. $\sigma_{\phi\sim-+}$ for clockwise source structure; $\sigma_{\phi\sim--}$ for counterclockwise source structure; $\sigma_{\phi\sim-0}$ for non-rotational source structure; $\sigma_{\phi\sim++}$ for clockwise sink structure; $\sigma_{\phi\sim+-}$ for counterclockwise sink structure; and $\sigma_{\phi\sim+0}$ for non-rotational sink structure, are assigned. Since this structure is the first determined root, a tree representation with the structures extracted in (S80) and (S90) as child nodes are obtained, and the algorithm ends.

(S120) The root structure is a flow on a spherical surface having 0-bundled ss-saddle at infinity. Of these, if there are s (s ≧ 0) orbits with a class-$\infty_{\sim\pm}$ structure that degenerates at the boundary of $\Omega$ in (S20) or a class-a structure, they are represented by a triplet COT representation of $\square_{a\infty\sim\pm\ s}$ and the structure is deleted until they disappear. The root structure $s_{\phi0}$ becomes the root, whose child nodes are represented as the triplets, and the corresponding tree representation can be obtained by connecting the three sets of structures extracted in (S80) and (S90) as its child nodes, and the algorithm ends.

(S130) The root structure is a spherical flow having an n-bundled ss-saddle at infinity. The number n of saddles existing inside the region of interest $\Omega$ is counted. Next, the uniform flow having a triplet $\square_{a\infty\sim\pm}$ consisting of the structure of class-$\infty_{\sim\pm}$ and class-a is removed. The root structure $s_{\phi n}$ becomes the root whose child nodes are represented as triplets, and the corresponding tree representation is obtained by connecting the three sets of structures extracted in (S80) and (S90), and the algorithm ends.

[String algorithm and application example]

Based on the classification theory and the allocation of the COT representation described above, the COT representation and the algorithm for assigning the tree structure associated therewith to the topological structure of the orbit group of the given intraventricular blood flow image data are constructed. The theory is given as a classification theory of flow of fit type on a sphere with an n-bundled ss-saddle, but when actually assigning a COT representation, it is more convenient to apply this algorithm for images with bounded region of interest $\Omega$. As a basic policy, first, the ss-saddle connection diagram in the region of interest $\Omega$ is constructed from the orbit group data of the given intraventricular blood flow image. Next, a character string is assigned from a small structure called the "innermost structure", and a larger structure containing it is inductively extracted. Compared to previous studies, the structure to be extracted is assumed to have no physical boundaries, so there are fewer types of structures to consider, but the basic policy is exactly the same. On the other hand, note that as a result of applying this method, the orbit structure that crosses the boundary $\partial\Omega$ of the region of interest transversely remains unextracted. Therefore, next, these orbit structures are extracted and a COT representation is assigned. Eventually, the root is reached, which completes the characterizations and extraction of discrete combination structures such as tree structures.

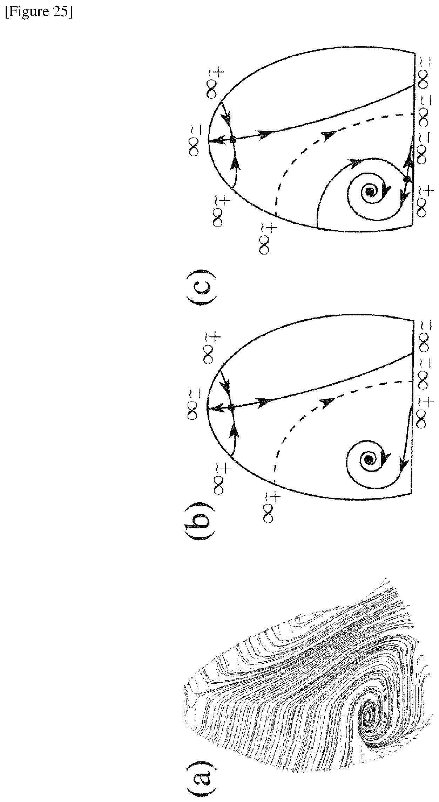

Here, with reference to FIG. 25, topological preconditioning of intraventricular blood flow image data will be described. For example, consider the left chamber blood flow image data (flow pattern) in the echocardiography VFM as shown in FIG. 25(a). Consider constructing the ss-saddle connection diagram from this flow. First, there is a clockwise sink flow structure on the left side of the region of interest, and a saddle domain is found at the top. There is no other characteristic structure. If an attempt is made to extract ss-saddle separatrix only from this information, a characteristic orbit structure as shown in FIG. **25**(b) is obtained as a topological data structure. However, this flow field is in topological contradiction because the direction of the uniform flow drawn by the dotted line in the figure and the direction of the flow structure created by the source structure are opposite. In order to solve such a situation, it is necessary to assume some structure outside the region of interest and correct topologically, but such a method is arbitrary. Therefore, in order to remove this arbitrariness in the method of correction, only "the operation of adding one saddle and connecting the ss-saddle separatrix consistently" is allowed. In this case, by adding one slidable saddle, and three ss-separatrix extending from the source structure to the saddle and three ss-saddle separatrices crossing the boundary transversely from the saddle, a flow structure that does not contradict the direction of the uniform flow of the dotted line can be constructed as shown in FIG. **25**(c). In fact, a saddle-like flow structure is found in the lower part of FIG. **25**(a).

The operation of eliminating the topological contradiction by adding a saddle to the outside is called topological preconditioning of the intraventricular blood flow image data. In the previous example, the determination whether or not to perform this pretreatment may be made by paying attention to the inside of the area surrounded by the flow field as shown by the dotted line.

[Conversion from ss-saddle connection diagram to COT representation]

First, the "innermost structure" is extracted from the ss-saddle connection diagram constructed by performing topological preconditioning. Here, the innermost structure refers to a flow structure having no structure inside. Since it is assumed that there is no physical boundary inside the region of interest in the orbit group data of the blood flow image in the ventricle, the innermost structure can be a zero-dimensional singular point structure, that is, a vortex center, a sink, or, a source. Based on this, the algorithm is configured as follows.

(Step 1) The innermost 0-dimensional singularity structure is searched from the ss-saddle connection diagram in the region of interest $\Omega$. COT representation with $\sigma_{\sim++}$ for source structure with counterclockwise rotation; $\sigma_{\sim+-}$ for source structure with clockwise rotation; $\sigma_{\sim+0}$ for source structure with no rotation; $\sigma_{\sim-+}$ for sink structure with counterclockwise rotation; $\sigma_{\sim--}$ for sink structure with clockwise rotation; $\sigma_{\sim-0}$ for sink structure with no rotation; $\sigma_+$ for vortex center with counterclockwise rotation; and $\sigma_-$ for vortex center with clockwise rotation are assigned. After assigning the COT representation, remove the structure from the orbit structure and replace it with the label $\square$. By replacing the label in this way, and by representing that the structure "not to have" inside structure, it is possible to convert the upper structure including the extracted structure into the innermost structure. This operation is continued until all 0-dimensional structures are deleted.

(Step 2) Next, the innermost (that is, labeled as $\square$ without a structure inside) one-dimensional structure and two-dimensional structure are searched from the ss-saddle connection diagram in the region of interest $\Omega$. These are given in COT representation, any of $p_\pm$, $p_{\sim\pm}$, $b_{\pm\pm}$, $b_{\pm\mp\pm}$, $b_\pm$, $b_{\sim\pm}$, $a_{\sim\pm}$, $q_{\sim\pm}$, $a_\pm$, $q_\pm$. If these structures are found, remove them from $\Omega$ and replace them with $\square$, which indicates that there is no structure inside. Since in the structure extracted in this situation, there is an internal orbit structure that was once removed and replaced with $\square$, an edge is generated, so that internal orbit structure is regarded as its own "child node" and the internal orbit structure itself is referred to as "parent". This operation is inductively continued until all the innermost structures are removed.

(Step 3) As a result of (Step 2), all the orbit structures are removed from the inside of the $\Omega$, but if there is no saddle connected to the n-bundled-ss-saddle inside the $\Omega$, the root structure is $\sigma_{\phi\sim\pm\pm}$, with only one source / sink, or the root domain $s_{\phi 0}$ consisting of a uniform flow that crosses the boundary of the region of interest. In the former case, the process proceeds to (step 4), and in the latter case, the process proceeds to (step 5). If saddle exists inside the $\Omega$, the root structure has an n-bundled ss-saddle (n $\geqq$ 1), so the process proceeds to (step 6).

(Step 4) The root structure is a flow on a spherical surface having no degenerated singularity. In this situation, when there is a source structure inside $\Omega$, the infinity becomes a sink. Assuming that the direction of rotation is opposite to the direction of rotation of the internal structure, the COT representations are $\sigma_{\phi\sim--}$ ($\square_{b\phi\sim+}$) (internal structure is a counterclockwise orbit); $\sigma_{\phi\sim-+}$ ($\square_{b\phi\sim+}$) (Internal structure is clockwise orbit); and $\sigma_{\phi\sim+0}$ ($\square_{b\phi\sim+}$) (Internal structure is non-rotating orbit). On the contrary, when the inside of $\Omega$ has a sink structure, the infinity becomes a source. The direction of rotation is also determined in the same way, and the COT representations are $\sigma_{\phi\sim+-}$ ($\square_{b\phi\sim-}$) (internal structure is counterclockwise orbit); $\sigma_{\phi\sim++}$ ($\square_{b\phi\sim-}$) (internal structure is clockwise orbit); and $\sigma_{\phi\sim+0}$ ($\square_{b\phi\sim-}$) (internal structure is a non-rotating orbit). However, $\square_{b\phi\sim\pm}$ contains the COT representation of all the structures extracted in (step 2), and a tree representation with this structure as the root and the structure extracted in (step 2) as child nodes is obtained, and the algorithm ends.

(Step 5) The root structure is a flow on a spherical surface with a 0-bundled ss-saddle at infinity. The inside of $\Omega$ has a uniform flow structure that crosses the boundary $\partial\Omega$ transversely. Among them, if there are s (s $\geqq$ 0) orbits having a class-$\infty_{\sim\pm}$ structure that degenerates to the boundary in step 2 or a structure of class-a, they are represented by a triplet COT $\square_{a\infty\sim\pm s}$, and a COT representation can be obtained by ordering them in order from the top under $s_{\phi 0}$ as shown in FIG. 15(b). Also, by using this structure as the root and connecting the triplet structure as its child nodes, the corresponding tree representation can be obtained, and the algorithm ends.

(Step 6) The root structure is a spherical flow having an n-bundled ss-saddle at infinity, wherein n is the number of saddles present in $\Omega$. In this situation, 3n + 1 domains are formed, which are divided by the 4n saddle separatrices from these n saddles, crossing $\partial\Omega$ transversely, and each of them consists of the structures of class-$\infty_{\sim\pm}$ and class-a. Since there is a uniform flow having a triplet $\square_{a\infty\sim\pm}$, a COT representation can be obtained by arranging them according to the rules as shown in FIGs. 15(c) and 15(d). Also, by using this structure as the root and connecting these triplets as its child structures, the corresponding tree representation can be obtained and the algorithm ends.

Using the above algorithm, the tree structure that accompanies the COT representation to the given flow structure does not have a one-to-one correspondence with the flow, but has a many-to-one correspondence. This is the same reason discussed in previous studies. In order to make this a one-to-one correspondence, in addition to the COT representation, a link structure indicating the connection status of the ss-saddle connection or saddle connection of the flow should be provided. The proof is exactly the same as in the previous research from the way of giving the COT representation and the way of giving the link structure, but as a result, the following theorem holds.

(Theorem 2) The flow of finite type on a sphere with a degenerated singularity n-bundled ss-saddle has a one-to-one correspondence with the COT representation and link structure composed of it.

How to give this COT representation will be described with reference to the example of FIG. FIG. 26(a) is orbit group data of intracardiac blood flow visualized by echocardiography VFM. This is because the papillary muscles on both sides near the apex of the heart begin to contract during the left ventricular systole, the blood flow obtains fluid dynamical balanced collided point at the saddle, and smooth ejection blood flow is enabled from the vortex at the basal portion of the left ventricle. This pattern is a typical blood flow pattern in the early stage of left ventricular systole, and is particularly referred to as "pattern A", because it has physiologically important meaning.

FIG. 26(b) is an ss-saddle connection diagram (topological data structure) extracted from FIG. 26(a) by performing the above-mentioned topological preconditioning. From here, the COT representation is given using the algorithm described above. First, by (step 1), the innermost singular point of the region of interest $\Omega$ is detected. Since there is one clockwise sink in the lower left, if the COT representation of $\sigma_{\sim--}$ is assigned to this and replaced with $\square$ indicating that the structure is removed, the structure is as shown in FIG. 26(c). At this point, there is no internal structure of $\Omega$ that must be extracted in step 2, so when looking for a structure of class-$a_{\infty\sim\pm}$ that degenerates to the infinity, there is a slidable saddle that connects the sink structure $\square$ and $\infty_{\sim-}$ at the bottom of the boundary, corresponding COT representation is assigned, degenerated into the boundary, and replaced with $\square_{\infty\sim-}$. In this situation, since the $\square$ of the structure contains the source extracted with (Step 1) as a "child" structure, note that $\square_{\infty\sim-}: = a_{\sim}\{\sigma_{\sim--}, \infty_{\sim-}\}$. If all of these orbit structures are extracted, only one saddle in which all separatrices cross the boundary $\partial\Omega$ transversely in the region of interest remains as shown in FIG. 26(d). Therefore, it can be seen that the root structure of this flow is $s_{\varphi1}$. To assign a COT representation to this, ss-saddle separatrix is numbered as $\infty^1_{\sim+}$, $\infty^2_{\sim-}$, $\infty^3_{\sim+}$, $\infty^4_{\sim-}$. In this situation, since there is one class-a $\infty_{\sim\pm}$ structure that degenerate to the infinity only in the domain divided by the separatrix of $\infty^1_{\sim+}$, and $\infty^2_{\sim-}$, a triplet [$\infty_{\sim+}, \lambda_{\sim}, a_{\sim-}\{\sigma_{\sim--}, \infty_{\sim-}\}$] is made, and embedded in the structure of the root area $s_{\varphi.1}$, and COT representation of the flow is given by

$$s_{\phi1}\ \{[\infty_{\sim+}, \lambda_\sim, a_{\sim-}\{\sigma_{\sim--}, \infty_{\sim-}\}], \lambda_\sim, \lambda_\sim, \lambda_\sim\}$$

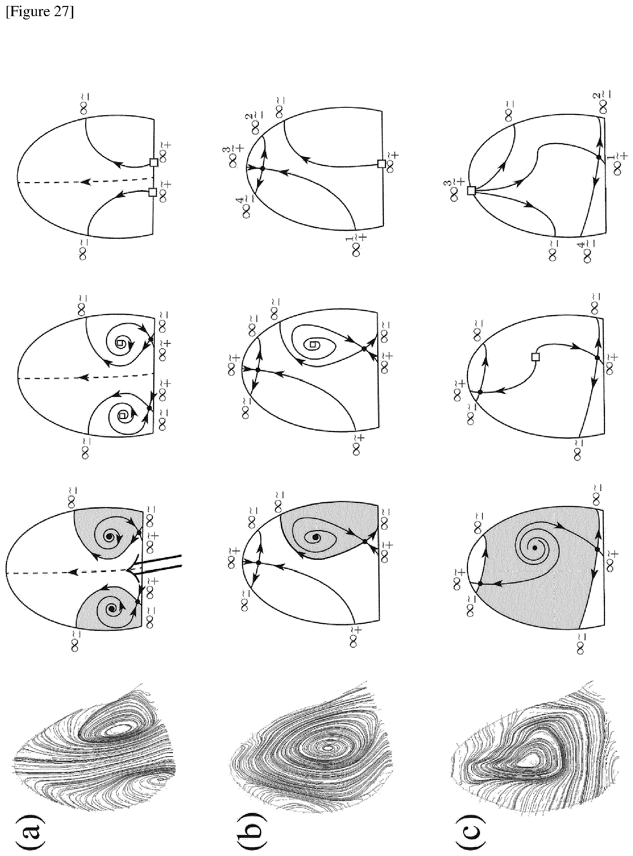

A similar procedure is used to assign COT representations for other patterns. In FIG. 27(a), the mitral valve inflow blood flow reaches its peak, and a vortex ring is formed on the back of the anterior-posterior leaflet due to the flow detachment, and a twin-shaped vortex flow is generated in the apex long-axis cross-section. This is a typical left ventricular diastolic blood flow orbit pattern, which is called "pattern B". FIG. 27(b) shows a typical vortex of the time phase in which a large intracardiac vortex is formed at the end of left ventricular diastole and a vortex is generated as if preparing smooth blood flow for the upcoming systole. This is called "pattern C". FIG. 27(c) is a typical corruption of two-dimensional flow inside the cross-sectional plane, often seen in late left ventricular dilatation, which is referred to as "pattern D". First, pattern B in FIG. 27(a) is a situation in which a uniform flow flows from bottom to top in the region of interest. As a result, a rotating flow is generated from side to side. From the configuration of the flow field, it can be seen that this is a "source". This is schematically shown on the right when the ss-saddle connection diagram is used. On the other hand, first, there is a counterclockwise source (COT representation $\sigma_{\sim++}$) on the left side and a clockwise source (COT representation $\sigma_{\sim+-}$) on the right side. By replacing, and further degenerating the two structures of class-$\infty_{\sim\pm}$ (COT representation is $a_{\sim+}\{\square, \infty_{\sim\pm}\}$) to the infinity to make $\square_{\infty\sim+}$, the root structure with a uniform flow of the two classes-$a\infty_{\sim\pm}$ results in a flow of $S_{\varphi0}$. Therefore, the COT representation of this flow is given below.

$$s_{\phi0}\ ([a_{\sim+}\{\sigma_{\sim++}, \infty_{\sim+}\}, \lambda_\sim, \infty_{\sim-}], [a_{\sim+}\{\sigma_{\sim+-}, \infty_{\sim+}\}, \lambda_\sim, \infty_{\sim-}])$$

Next, in the pattern C of FIG. 27(b), there is a large source vortex structure on the right side of the region of interest, and a small saddle domain exists on the upper left side; thus, it is written as a connection diagram, and one clockwise source $\sigma_{\sim+-}$ is extracted from it. Furthermore, if the structure $a_{\sim+}\{\square, \infty_{\sim+}\}$ of class -$\infty_{\sim+}$ is degenerated to the boundary, only one saddle remains in the region of interest, so the root structure is $s_{\varphi1}$. By assigning numbers $\infty 1_{\sim+}$ to $\infty^4_{\sim-}$ to the separatrix connecting from this saddle to the boundary and by arranging the internal structures of the structures divided by those saddles in the order, the COT representation becomes as follows.

$$s_{\phi1}\ \{[a_{\sim+}\{\sigma_{\sim+-}, \infty_{\sim+}\}, \lambda_\sim, \infty_{\sim-}], \lambda_\sim, \lambda_\sim, \lambda_\sim\}$$

Finally, in the pattern D of FIG. 27 (c), a large vortex structure can be seen in the center, and saddles are present in the upper left and lower right so as to sandwich the large vortex structure. Since the inside of this vortex structure is white, it is possible that some internal structure is contained here, but here, assuming that the source is in the center, the ss-saddle connection diagram is written. First, if this clockwise source point ($\sigma_{\sim+-}$) is extracted and replaced with $\square$, it can be seen that there are two ss-components connecting this source structure and the upper and lower saddles. In principle, one of these saddles, this source structure, and the source structure $\infty_{\sim+}$ at infinity are regarded as one class-$\infty_{\sim+}$ structure and is degenerate to the infinity, and there is an option to degenerate the structure into the source above the boundary or to degenerate the structure into the source below the boundary. In such a case, if you decide to always degenerate the upward one and actually degenerate the structure into the boundary, there will be only one saddle remaining in the region of interest, and the root structure is $s_{\varphi 1}$, and the rest will be degenerated to make the three sets corresponding to two of the orbit of the class-a$\infty_{\sim\pm}$ out of the structure, the crossing point with the ss-saddle separatrix and the boundary connecting to the saddle are numbered $\infty^1_{\sim+}$ to $\infty^4_{\sim-}$, and sequentially arranged. Then the following COT representation is obtained.

$$s_{\phi 1}\{\lambda_\sim, [a_{\sim+} \{\sigma_{\sim+-}, \infty_{\sim+}\}, \lambda_\sim, \infty_{\sim-}], [a_{\sim+} \{\sigma_{\sim+-}, \infty_{\sim+}\}, \lambda_\sim, \infty_{\sim-}], \lambda_\sim\}$$

[Application to cardiovascular echo data of healthy subjects]

Hereinafter, an example in which the above-mentioned technique is applied to the cardiovascular echo data of a healthy person will be described. Specifically, a COT representation is assigned to the ss-saddle connection diagram obtained by performing topological preconditioning on the intraventricular blood flow image data of a healthy person during one beat, which was accurately acquired by shortening the measurement time interval.. As a result, it is shown that the above-mentioned patterns A to D appear as characteristic flow patterns appearing in the heartbeat. Furthermore, basic medical consideration will be added to the characteristics of the state of the cardiovascular system, such as the relationship with the cardiac function and which phase in cardiac cycle should be focused on in the character string representation of the intraventricular blood flow image data in the following phases.

Here, the orbit group data (specifically, image data consisting of 0 to 44 frames) of the intraventricular blood flow image measured during one heartbeat is used. By performing topological preconditioning on these frames, an ss-saddle connection diagram can be configured for each frame.

By observing these frames, it can be seen that there are four phases in a cardiac cycle in the heart where the characteristic orbit topological structure with high blood flow velocity can be seen.

- Mid-Systolic: Blood flow is ejected with strong force from the lower right of the region of interest. It represents the orbit structure of the phase in which blood flow is ejected during one cardiac cycle.
- Rapid fling diastolic: Blood flow is injected with strong force from around the center of the region of interest. It represents the orbit structure of the phase in which blood flow flows during one cardiac cycle.
- Late diastole: The inflow and outflow of blood flow to the region of interest is stopped, the left side vortex generated by the inflow is dissipated, and the large right vortex structure is maintained.
- End diastole: Represents the orbit structure of the phase in which the right side vortex structure sustained in the previous phase moves to the center due to its inertia.

Hereinafter, the ss-saddle connection diagram is shown for the orbit group of each phase, and the COT representation is given to the ss-saddle connection diagram to describe the phase structure of the flow.

(Left ventricular systole)

During systole, the orbit shape changes but the topological structure does not. A strong blood flow from the lower right to the outside of the boundary is observed in the area of interest. As a result, one saddle with ss-saddle separatrix connected to the point at infinity is observed in the upper part of the region of interest, and one large clockwise sink rotating flow structure exists on the left side of the region of interest. As a result of these topological pretreatments, one saddle associated with this structure has been added, which is the structure of pattern A shown in FIG. 26. The COT representation in this phase is given below.

$$s_{\phi 1} \{\lambda_\sim, \lambda_\sim, \lambda_\sim, [\infty_{\sim+}, \lambda_\sim, a_{\sim-}\{\sigma_{\sim--}, \infty_{\sim-}\}]\}$$

This COT representation seems to be different from the one given to the above-mentioned pattern A, but it is the same representation because there is a cyclic option in the selection method of ss-saddle separatrix in the root structure $s_{\varphi 1}$. This region does not completely overlap with the negative domain of vorticity, but it is a topological extraction of the rotational flow structure. Hereinafter, such a structure is referred to as a "topological vortex structure". The existence of such a structure is expressed by the triplet $[\infty_{\sim+}, \lambda_\sim, a_\sim \{\sigma_{\sim-}, \infty_{\sim-}\}]$ in the COT representation, including the direction of rotation.

(At the time of left ventricular diastolic inflow)

When the outflow stops in the previous phase, the aortic valve is closed but the mitral valve is not yet open.

After that, when the mitral valve is open and the left ventricular diastolic inflow phase begins, flow with a strong signal enters into the inside of the region of interest, and the structure of the flow becomes clear. First, the upper saddle seen in the outflow phase disappears, so the root structure changes to $s_{\varphi 0}$. Immediately after entering the inflow phase, a large counterclockwise outflow topological vortex structure is formed on the left side of the region of interest with the flow. The COT representation in this situation is as follows.

$$s_{\phi 0}\ ([a_{\sim+}\{\sigma_{\sim++},\ \infty_{\sim+}\},\ \lambda_{\sim},\ \infty_{\sim-}])$$

After this, the inflow with strong force continues, and another new clockwise outflow topological vortex structure is formed on the right side, resulting in a characteristic "twin vortex" structure. This is pattern B shown in FIG. 27(a), the COT representation of which is given below.

$$s_{\phi 0}\ ([a_{\sim+}\{\sigma_{\sim++}\ ,\ \infty_{\sim+}\},\ \lambda_{\sim}\ ,\ \infty_{\sim-}],\ [a_{\sim+}\{\sigma_{\sim+-},\ \infty_{\sim+}\ \},\ \lambda_{\sim},\ \infty_{\sim-}])$$

In this way, it is shown that the number of triplets increases as the number of source vortex structures increases, and each triplet extracts the topological vortex structure.

(Late diastole)

When the inflow stops, the vortex structure on the left side dissipates and disappears, but the source clockwise vortex structure on the right side remains large and continues to exist. As a result, the transition to the flow of the pattern C having the ss-saddle connection diagram occurs. The COT representation is as follows, as already given.

$$s_{\phi 1}\ \{[a_{\sim+}\{\sigma_{\sim+},\ \infty_{\sim+}\},\ \lambda_{\sim},\ \infty_{\sim-}],\ \lambda_{\sim},\ \lambda_{\sim},\ \lambda_{\sim}\}$$

The clockwise topological vortex source structure represented by this triplet begins to move upward little by little while maintaining its size in this phase.

(End diastole)

The large clockwise topological source vortex structure seen on the right side in the previous phase moves to the center of the region of interest due to its inertia, while gradually reducing its size due to the viscous dissipation of the vortex. As a result, the distance between the outward ss-saddle connected to the saddle and the ss-saddle separatrix at the boundary of the vortex structure domain, which was seen in the previous phase, becomes short. At some point, they coincide and a transition occurs in the topological structure, and the clockwise sink vortex structure changes to pattern D surrounded by two saddle separatrices. This topological vortex domain is a domain painted in gray in the center of the region of interest. This COT representation is as follows, as already given.

$$s_{\phi 1}\ \{\lambda_{\sim},\ [a_{\sim+}\ \{\sigma_{\sim+-},\ \infty_{\sim+}\},\ \lambda_{\sim},\ \infty_{\sim-}],\ [a_{\sim+}\ \{\sigma_{\sim+-},\ \infty_{\sim+}\},\ \lambda_{\sim},\ \infty_{\sim-}],\ \lambda_{\sim}\ \}$$

Note that even if the root structure changes, the represented triplet structure and its order do not change.

[Comparison with heart failure data]

Next, regarding how the intracardiac vortex patterns differ between healthy cases and heart failure cases, the difference is examined by actually giving a COT representation. Here, since the analysis is too enormous to describe the changes in the pattern of vortex flow in all phases in various diseases, a sample of severe heart failure cases of dilated cardiomyopathy, which is a typical disease among heart failure cases is analyzed. In heart failure, the essence of the condition is that the amount of blood ejected from the left ventricle does not meet the oxygen demand of systemic organs. Therefore, in this study, we first focused on the systole in which the left ventricle pumps blood, and in the case of severe heart failure due to dilated cardiomyopathy from the early to middle systole in which the above-mentioned pattern A is likely to appear clearly, we will examine how the COT representation differs from that of healthy patients. These cases of dilated cardiomyopathy were cases in which severe left ventricular hypokinesia and impaired left ventricular ejection fraction were observed, and the left heart assist device had to be attached due to severe heart failure.

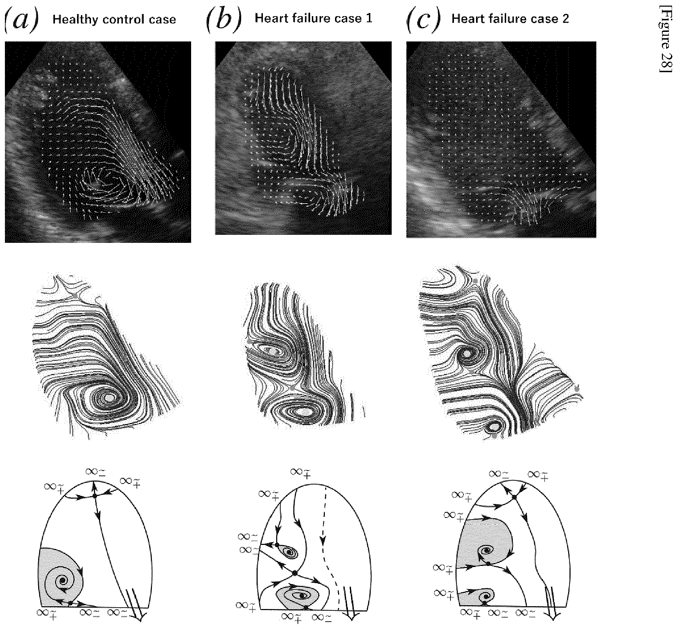

FIG. 28 is a streamline visualization of the left ventricular intracardiac blood flow in the early to middle contractions visualized by echocardiography VFM (vector flow mapping), that is, the phase when the aortic valve begins to open. (a) shows an example of a healthy volunteer, (b) shows an example of a heart failure patient 1, and (c) shows an example of a heart failure patient 2. The upper figure is a superposition of the blood flow velocity vector on the echocardiography image. The middle figure shows only streamlines extracted from the upper image to clearly show their singular points, and shows the orbit group data in the region of interest. The position of the singular point is calculated numerically. The degenerated singular point, which is the boundary of the myocardial wall, is not shown. The lower figure shows the streamlined structures extracted from the orbit group data in the middle stage, and the ss-saddle connection diagram is given to them.

First, in a healthy case, as described above, a COT representation corresponding to the following pattern A can be obtained.

$$s_{\phi 1}\{[\infty_{\sim+}, \lambda_{\sim}, a_{\sim}\{\sigma_{\sim\sim}, \infty_{\sim}\}], \lambda_{\sim}, \lambda_{\sim}, \lambda_{\sim}\}$$

In this pattern, there is a vortex on the posterior wall side of the basal part of the left ventricle. The flow has configuration in which blood ejection can be smoothly performed from this vortex toward the aortic valve, which is considered to be a rational flow. The vortex domain represented by the triplet $[\infty_{\sim+}, \lambda_{\sim}, a_{\sim}\{\sigma_{\sim\sim}, \infty_{\sim}\}]$ is characterized as a gray domain in the ss-saddle connection diagram, and is characterized as a gray domain at the base of the heart. We have succeeded in clarifying the extent of the clockwise sink vortex domain formed on the posterior wall side.

Next, in "Heart failure example 1", when looking at the streamlines in the same systolic phase, it can be seen that the patterns are clearly different and the pattern A is broken. In fact, the COT representation of this phase of this case is as follows.

$$s_{\phi 1}\{[\infty_{\sim+}, \lambda_{\sim}, a_{\sim}\{\sigma_{\sim\sim}, \infty_{\sim}\}], \lambda_{\sim}, [\infty_{\sim+}, a_{\sim}(b_{\sim+}(\sigma_{\sim+\sim}, \lambda_{\sim})), \infty_{\sim}], \lambda_{\sim}\}$$

In this case, the $a_{\sim}\{\sigma_{\sim\sim}, \infty_{\sim}\}$ vortex structures appeared in the apex of the divided domains by the saddle separatrix, would not be expected to be used effectively as an output drive. Further, a vortex having a doubly connected vortex structure of $b_{\sim+}$ appears on the basal side through a domain of $\lambda_{\sim}$ without a vortex on the lateral wall side. For this reason, it is suggested that the blood flow is not smoothly ejected from the vortex to the aorta as a whole, but the vortex is hard to break and there is a high possibility that blood remains in the heart. The COT representation reveals a doubly connected vortex structure domain formed on the basal side. In these triplets, the lower vortex structure is represented as $[\infty_{\sim+}, \lambda_{\sim}, a_{\sim}\{\sigma_{\sim\sim}, \infty_{\sim}\}]$, and the upper vortex structure, $[\infty_{\sim+}, a_{\sim}(b_{\sim+}(\sigma_{\sim+\sim}, \lambda_{\sim})), \infty_{\sim}]$. It goes without saying that the difference in pattern clearly appears as a difference in COT representation, and although the lower vortex structure originally corresponds to the vortex structure in healthy cases, the structure is not sufficiently formed due to cardiac dysfunction. As a result, a vortex domain confining the small vortex structure on the upper side is formed, and the area of the corresponding vortex domain is also very small. In addition, the saddle position of the ss-saddle separatrix connected to the infinity corresponding to the root structure $s_{\phi 1}$ that separates these triplets has also moved to the lower left side, which makes a remarkable difference in the flow structure from the healthy cases.

Next, in "Heart Failure Case 2" as well, when looking at the streamlines in the same systolic phase, it can be seen that the patterns are clearly different and the pattern A is broken. The COT representation of this phase of this case is as follows.

$$s_{\phi 1}\{[\infty_{\sim+}, \lambda_{\sim}, a_{\sim}\{\sigma_{\sim\sim}, \infty_{\sim}\}] \cdot [\infty_{\sim+}, \lambda_{\sim}, a_{\sim}\{\sigma_{\sim\sim}, \infty_{\sim}\}], \lambda_{\sim}, \lambda_{\sim}, \lambda_{\sim}\}$$

In this vortex, $a_{\sim}\{\sigma_{\sim\sim}, \infty_{\sim}\}$ structures in the basal portion seen in a pattern A, form the doubly connected vortex structure, and a large vortex appears in the mid portion of the ventricle distant from the aortic valve. Therefore, it can be seen that the vortex is not used effectively for blood ejection. In this heart failure case, the position of ss-saddle separatrix leading to the infinity that determines the root structure $s_{\phi 1}$ is close to that of healthy cases, but the formation of the vortex domain at the basal portion of the heart is inadequate, so COT representation of the vortex structure of duplicate clockwise sink is observed that is connected to the same triplet $[\infty_{\sim+}, \lambda_{\sim}, a_{\sim}\{\sigma_{\sim\sim}, \infty_{\sim}\}]$. That is, the vortex structure of the pattern A, which should originally be formed by one, is separated into two. As a result, the vortex flow domains corresponding to each are also small.

It is clear that the morphological features of the streamlines are different only from the images. However, according to the present embodiment, this difference is not an ambiguous index of a mere image difference, but appears in a clearly different form in the COT representation as a difference in the vortex structure represented by the streamline topological structure. This serves as an identifier that holds the physical information of the flow state. Not only that, by analyzing this, it is possible to simultaneously extract quantitative information such as the separation of the compressible domain and the incompressible domain as the vector field of fluid represented by the COT representation, and to quantify their existence domain. In this point, it is important to be able to simultaneously represent qualitative and quantitative information when considering cardiac function.

Industrial Availability

This invention is applicable to word representation devices, word representation methods, and programs for flow patterns.

EXPLANATION OF NUMERALS

1 ... word representation device, 2 ... word representation device, 10 ... storage unit, 21 ... root determination means, 22 ... tree representation construction means, 23 ... COT representation generation means, 24 ... combination structure extraction means, S1 ... A step to determine the root, S2 ... A step to construct a tree representation, S3 ... A step to generate a COT representation

**Claims**

1.  A word representation device for representing a streamline structure of a flow pattern in a two-dimensional domain in words, the device comprising a storage unit and a word representation generation unit,

    wherein the storage unit stores the correspondence relationship between each streamline structures and its character corresponding to a plurality of streamline structure constituting the flow pattern, and
    wherein, the word representation generation unit comprises a rout determining means, a tree representation construction means, and a COT representation generation means, wherein

    the root determining means determines the root of a given flow pattern;
    the tree representation construction means extracts the streamline structure of the given flow pattern, assigns characters to the extracted streamline structure based on the correspondence relationship stored in the storage unit, and constructs the tree representation of the given flow pattern, by repeatedly executing the process of deleting the extracted streamline structure from the innermost part of the flow pattern until the root is reached; and
    the COT representation generation means converts the tree representation constructed by the tree representation constructing means into a COT representation to generate a word representation of the given flow pattern; and

    wherein a flow constituting the flow pattern includes a flow generated by moving physical boundaries.

2.  The word representation device according to claim 1, wherein the streamline structure constituting the flow pattern includes the root structure on a surface with an n-bundled ss-saddle degenerated singular point.

3.  The word representation device according to claim 1 or 2, wherein the flow pattern is a pattern of intraventricular blood flow.

4.  The word representation device according to any one of claims 1 to 3, further comprising a display unit for displaying a topological data structure extracted from the flow pattern.

5.  A word representation method for representing a streamline structure of a flow pattern in a two-dimensional domain in words, the method executed by a computer equipped with a storage unit and a word representation generation unit,

    wherein the storage unit stores the correspondence relationship between each streamline structures and its character corresponding to a plurality of streamline structure constituting the flow pattern, and
    wherein the word representation generation unit executes a root determining step, a tree representation step, and a COT representation generation step, wherein
    the root determining step determines the root of a given flow pattern;

the tree representation construction step extracts the streamline structure of the given flow pattern, assigns characters to the extracted streamline structure based on the correspondence relationship stored in the storage unit, and constructs the tree representation of the given flow pattern, by repeatedly executing the process of deleting the extracted streamline structure from the innermost part of the flow pattern until the root is reached; and the COT representation generation step converts the tree representation constructed by the tree representation constructing step into a COT representation to generate a word representation of the given flow pattern; and wherein a flow constituting the flow pattern includes a flow generated by moving physical boundaries.

6. A program for a process executed by computer equipped with a storage unit and a word representation generation unit,

wherein the storage unit stores the correspondence relationship between each streamline structures and its character corresponding to a plurality of streamline structure constituting the flow pattern, and wherein the word representation generation unit executes

a root determining step determining the root of a given flow pattern;
a tree representation construction step extracting the streamline structure of the given flow pattern, assigning characters to the extracted streamline structure based on the correspondence relationship stored in the storage unit, and constructing the tree representation of the given flow pattern, by repeatedly executing the process of deleting the extracted streamline structure from the innermost part of the flow pattern until the root is reached; and
a COT representation generation step converting the tree representation constructed by the tree representation constructing step into a COT representation to generate a word representation of the given flow pattern; and

wherein a flow constituting the flow pattern includes a flow generated by moving physical boundaries.

[Figure 1]

[Figure 2]

[Figure 3]

[Figure 4]

[Figure 5]

[Figure 6]

[Figure 7]

[Figure 8]

(a) $\boxed{b_{\pm\pm}}$

(b) $\boxed{b_{\pm\mp}}$

$b_{++}$   $\square_{b_+}$   $\square_{b_+}$

$b_{--}$   $\square_{b_-}$   $\square_{b_-}$

$b_{+-}$   $\square_{b_-}$   $\square_{b_+}$

$b_{-+}$   $\square_{b_+}$   $\square_{b_-}$

[Figure 9]

[Figure 10]

[Figure 11]

[Figure 12]

EP 4 113 538 A1

[Figure 13]

[Figure 14]

[Figure 15]

[Figure 16]

[Figure 17]

Parent - - - - - - - - - - - - - - - - - - - - - - - - - - - - - - - - - - ● a

Child - - - - - - - - - - - - - - - - - - - - - - - - ● b     ○ h     ● i

Grand child (child's child) - - - - - ● c   ○ e   ● f     ○ j

Great-Grandchild (child's child's child) - - - - - - ○ d     ○ g

○ : Terminal vertex (leaf)

● : Non-terminal vertex

[Figure 18]

[Figure 19]

EP 4 113 538 A1

[Figure 20]

```
                    ┌─────────────────────┐
                    │        Start        │
                    └─────────────────────┘
                               │
                               ▼
                    ┌─────────────────────┐
                    │  Determine the Root │ ── S1
                    └─────────────────────┘
                               │
                               ▼
                    ┌─────────────────────┐
                    │ Construct the       │ ── S2
                    │ representation      │
                    └─────────────────────┘
                               │
                               ▼
                    ┌─────────────────────┐
                    │ Construct the tree  │ ── S3
                    │ representation      │
                    └─────────────────────┘
                               │
                               ▼
                    ┌─────────────────────┐
                    │         End         │
                    └─────────────────────┘
```

EP 4 113 538 A1

EP 4 113 538 A1

```
                    ┌──────────┐
                    │  Start   │
                    └────┬─────┘
                         │                    S10                              S30
                         ▼
              ╱╲                              ╱╲
            ╱    ╲      Need the topological    Y    ╱    ╲    Is the topological       N    ┌──────────────────┐
          ╱        ╲    preconditioning?   ─────────╱        ╲  preconditioning possible? ───│ End with an error │
            ╲    ╱                              ╲    ╱                                        └──────────────────┘
              ╲╱                                  ╲╱
               │ N                                 │ Y            S35
               │                                   ▼
               │                        ┌────────────────────────────────┐
               │                        │   topological preconditioning   │
               │                        └────────────────────────────────┘
               │◄───────────────────────────────────┘
               │                    S20
               ▼
    ┌────────────────────────┐
    │ Extraction of ss-saddle │
    │ connection diagram      │
    └────────────┬───────────┘
                 │
                 ▼
            ┌─────────┐
            │   End   │
            └─────────┘
```

[Figure 21]

[Figure 22]

```
          ┌─────────────┐
          │    Start    │
          └─────────────┘
                 │
                 ▼                    S40
   ┌──────────────────────────┐
   │ Extract saddles connected │
   │ to ss-component           │
   └──────────────────────────┘
                 │
                 ▼                    S50
   ┌──────────────────────────┐
   │ Extract ss-component      │
   │ connected to the saddle   │
   └──────────────────────────┘
                 │
                 ▼                    S60
   ┌──────────────────────────┐
   │ Extract ss-separatrix     │
   │ connected to the saddle   │
   └──────────────────────────┘
                 │
                 ▼                    S70
   ┌──────────────────────────┐
   │ Construct a plane graph   │
   └──────────────────────────┘
                 │
                 ▼
          ┌─────────────┐
          │     End     │
          └─────────────┘
```

[Figure 23(a)]

Start

S80

Extract the innermost zero-dimensional structures and assigns the corresponding COT representation.

S85

N ← Are all the point structures removed?

Y

S90

Extract innermost $p_{\pm}$, $p_{\sim\pm}$, $b_{\pm\pm}$, $b_{\pm\mp}$, $b_{\pm}$, $b_{\sim\pm}$, $a_{\sim\pm}$, $q_{\sim\pm}$, $a_{\pm}$, $q_{\pm}$ structures

S95

N ← Are all the innermost structures removed?

Y

A

[Figure 23(b)]

A

S130 — Determine the root structure as $s_{\Phi n}$, connect its triplet structure as a child node (S80), obtain the tree representation by connecting the tree structure extracted in (S90).

S100 — Are these saddles inside the domain connecting to the n-bundled ss-saddle?

Y

N

S105 — (Condition 1) The root structure has only one source/sink. (Condition 2) The root structure consists of uniform flow crossing the boundary of the domain transversely. (Either condition 1 or 2 should be always satisfied)

Condition 1

S120 — Obtain the tree representation in which the root structure $s_{\Phi 1}$ connected to the triplet structure as its child node, by connecting the tree structure extracted by (S80) and (S90) steps.

Condition 1

S110 — Obtain the tree representation in which the root structure of $\sigma_{\phi \sim -+}$, $\sigma_{\phi \sim --}$, $\sigma_{\phi \sim ++}$, $\sigma_{\phi \sim +-}$, or $\sigma_{\phi -+0}$ is the root, and the structure extracted by (S80) and (S90) steps is the child node.

End

[Figure 24]

[Figure 25]

[Figure 26]

[Figure 27]

[Figure 28]

**INTERNATIONAL SEARCH REPORT**

| International application No. |
| --- |
| PCT/JP2021/003206 |

**A. CLASSIFICATION OF SUBJECT MATTER**
G16Z 99/00(2019.01)i; G16H 30/00(2018.01)i
FI: G16Z99/00; G16H30/00

According to International Patent Classification (IPC) or to both national classification and IPC

**B. FIELDS SEARCHED**

Minimum documentation searched (classification system followed by classification symbols)
G16Z99/00; G16H30/00

| Documentation searched other than minimum documentation to the extent that such documents are included in the fields searched | |
| --- | --- |
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus (JDreamIII); JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| A | 宇田 智紀，パーシステントホモロジーとレーブグラフを用いた2次元ハミルトンベクトル場の流線位相構造の自動抽出アルゴリズム，日本応用数理学会論文誌, 25 June 2019, vol. 29, no. 2, pp. 187-224, entire text, all drawings, (UDA, Tomoki, "Algorithms Converting Streamline Topologies for 2D Hamiltonian Vector Fields Using Reeb Graphs and Persistent Homology", Transactions of the Japan Society for Industrial and Applied Mathematics) | 1-6 |
| A | WO 2016/072515 A1 (JAPAN SCIENCE AND TECHNOLOGY AGENCY) 12 May 2016 (2016-05-12) entire text, all drawings | 1-6 |

☒ Further documents are listed in the continuation of Box C.  ☒ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 02 April 2021 (02.04.2021) | 13 April 2021 (13.04.2021) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

| | International application No. |
|---|---|
| | PCT/JP2021/003206 |

C (Continuation).　DOCUMENTS CONSIDERED TO BE RELEVANT

| Category* | Citation of document, with indication, where appropriate, of the relevant passages | Relevant to claim No. |
|---|---|---|
| A | 坂上 貴之，二次元多重連結領域内における構造安定な非圧縮流れの文字列表現アルゴリズム，数理解析研究所講究録, June 2014, vol. 1900, pp. 11-25, entire text, all drawings, non-official translation (SAKAJO, Takashi, "A string representation algorithm for structurally stable incompressible flow within a two-dimensional multiply connected domain", RIMS Kokyuroku) | 1-6 |

Form PCT/ISA/210 (continuation of second sheet) (January 2015)

## INTERNATIONAL SEARCH REPORT
Information on patent family members

International application No.

PCT/JP2021/003206

| Patent Documents referred in the Report | Publication Date | Patent Family | Publication Date |
|---|---|---|---|
| WO 2016/072515 A1 | 12 May 2016 | US 2017/0323033 A1<br>EP 3217303 A1 | |

Form PCT/ISA/210 (patent family annex) (January 2015)

**EP 4 113 538 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014041917 A
- WO 2016072515 A
- WO 2013077013 A
- WO 2014185521 A